# EUROPEAN PATENT APPLICATION

(11) **EP 3 372 675 A1**
(43) Date of publication of application: **12.09.2018**
(21) Application number: 17160178.4
(22) Date of filing: 09.03.2017
(51) Int. Cl.: C12N 5/0786, A61K 35/15, C12N 15/117

(54) **METHODS FOR IN VITRO DIFFERENTIATION OF MONOCYTES TO REGULATORY MACROPHAGES**

(71) Applicant: Université de Liège, 4000 Liège (BE)
(72) Inventor: Bureau, Fabrice, 6043 Ransart (BE); Marichal, Thomas, 4861 Soiron (BE); Desmet, Christophe, 4020 Liège (BE); Sabatel, Catherine, 4550 Saint-Séverin (BE); Radermecker, Coraline, 5590 Ciney (BE); Fiévez, Laurence, 4210 Oteppe (BE); Olivier, Sabine, 4000 Liège (BE)
(74) Representative: De Clercq, Annelies

(57) **Abstract**

The present invention relates to a method for *in vitro* differentiation of monocytes to regulatory macrophages, comprising culturing the monocytes in a medium in the presence of an immunostimulatory nucleic acid. The present invention also provides regulatory macrophages obtainable or obtained by such method and their use in treating inflammatory respiratory diseases, in particular chronic respiratory diseases, such as asthma or chronic obstructive pulmonary disease.

SEQUENCE LISTING

<110>  Université de Liège

<120>  METHODS FOR IN VITRO DIFFERENTIATION OF MONOCYTES TO REGULATORY
       MACROPHAGES

<130>  ULG-015

<160>  14

<170>  PatentIn version 3.5

<210>  1
<211>  6
<212>  DNA
<213>  Artificial Sequence

<220>
<223>  CpG nucleic acid


<220>
<221>  misc_feature
<222>  (1)..(2)
<223>  n is a, c, g, or t

<220>
<221>  misc_feature
<222>  (5)..(6)
<223>  n is a, c, g, or t

<400>  1
nncgnn                                                                 6


<210>  2
<211>  8
<212>  DNA
<213>  Artificial Sequence

<220>
<223>  CpG nucleic acid


<220>
<221>  misc_feature
<222>  (1)..(1)
<223>  from 0 to 25 nucleotides

<220>
<221>  misc_feature
<222>  (2)..(3)
<223>  n is a, c, g, or t

<220>
<221>  misc_feature
<222>  (6)..(7)
<223>  n is a, c, g, or t

<220>
<221>  misc_feature
<222>  (8)..(8)

<223>  from 0 to 25 nucleotides

<400>  2
nnncgnnn                                                                 8


<210>  3
<211>  6
<212>  DNA
<213>  Artificial Sequence

<220>
<223>  exemplary human CpG motif

<400>  3
gtcgtt                                                                   6


<210>  4
<211>  6
<212>  DNA
<213>  Artificial Sequence

<220>
<223>  exemplary mouse CpG motif

<400>  4
gacgtt                                                                   6


<210>  5
<211>  20
<212>  DNA
<213>  Artificial Sequence

<220>
<223>  Mouse non-CpG DNA

<400>  5
gcttgatgac tcagccggaa                                                   20


<210>  6
<211>  20
<212>  DNA
<213>  Artificial Sequence

<220>
<223>  Mouse CpG DNA

<400>  6
tccatgacgt tcctgatgct                                                   20


<210>  7
<211>  21
<212>  DNA
<213>  Artificial Sequence

<220>
<223>  primer

<400>  7

agctgaacat gaacggcatc t                                                21

<210>  8
<211>  18
<212>  DNA
<213>  Artificial Sequence

<220>
<223>  primer

<400>  8
gatcggccag ccatctga                                                    18

<210>  9
<211>  20
<212>  DNA
<213>  Artificial Sequence

<220>
<223>  primer

<400>  9
agcccagtgt taccaccaag                                                  20

<210>  10
<211>  20
<212>  DNA
<213>  Artificial Sequence

<220>
<223>  primer

<400>  10
acccaagaac aagcacaagg                                                  20

<210>  11
<211>  19
<212>  DNA
<213>  Artificial Sequence

<220>
<223>  primer

<400>  11
catggctcgc tcggtgacc                                                   19

<210>  12
<211>  21
<212>  DNA
<213>  Artificial Sequence

<220>
<223>  primer

<400>  12
aatgtgaggc gggtggaact g                                                21

<210>  13
<211>  24
<212>  DNA
<213>  Artificial Sequence

<220>
<223>  primer

<400>  13
agctactgta atgatcagtc aacg                                              24


<210>  14
<211>  20
<212>  DNA
<213>  Artificial Sequence

<220>
<223>  primer

<400>  14
agaggtcctt ttcaccagca                                                   20

## Description

### FIELD OF THE INVENTION

The present invention is broadly in the medical field. In particular, the invention relates to methods of treating inflammatory respiratory diseases, in particular chronic respiratory diseases, such as asthma or chronic obstructive pulmonary disease (COPD), in a subject. The invention also concerns methods for *in vitro* differentiation of monocytes to regulatory macrophages, which can be used for the treatment (including therapeutic and/or preventative measures) of inflammatory respiratory diseases.

### BACKGROUND OF THE INVENTION

Dramatic increase in the prevalence of allergic asthma over the past decades points toward alterations in the environment. The hygiene hypothesis postulates that decreased exposure to environmental and commensal microbes or their products, due to improved life conditions, is responsible for this rise. In line with this, epidemiological studies have demonstrated that growing up on a farm, where exposure to bacterial endotoxin (lipopolysaccharides; LPS) is high, reduces the risk of allergic sensitization. However, whether LPS mediates the aforementioned protective effects is uncertain. Indeed, concurrent studies in non-rural environments have suggested that exposure to LPS is rather a risk factor for increased asthma prevalence and severity. Moreover, although LPS has demonstrated protective effects in some animal models, it is generally accepted that it promotes experimental asthma.

Another candidate for mediating the protective effects of microbe-rich environments is bacterial DNA, which contains unmethylated CpG motifs with immunomodulatory properties (Krieg, 2006, Nat. Rev. Drug Discov., 5, 471-484). Bacterial DNA, like LPS, is present at high levels in dust from rural homes, and treatment with synthetic CpG nucleic acids consistently prevents allergic sensitization in animal models. Moreover, CpG nucleic acids are able to reverse established asthma in animals, and has shown efficacy in attenuating allergic disorders in human clinical trials (Beeh et al., 2013, J. Allergy Clin. Immunol., 131, 866-874; Creticos et al., 2006, N. Engl. J. Med., 355, 1445-1455; Krieg, 2006, *supra*). How CpG nucleic acid exerts these unique immunomodulatory effects remains unclear. It has first been proposed that CpG nucleic acid, which is often co-administered with allergens, redirects allergen-specific T helper type 2 (Th2) cell responses towards Th1 or regulatory T cell responses (Broide et al., 1998, J. Immunol., 161, 7054-7062; Kline et al., 2002, Am. J. Physiol. Lung Cell. Mol. Physiol., 283, L170-179; Kline et al., 1998, J. Immunol., 160, 2555-2559; Krieg, 2012, Nucleic Acid Ther., 22, 77-89). However, it is now clear that CpG nucleic acid is able of conferring protection in the absence of allergens (Beeh et al., 2013, *supra*; Broide et al., 1998, *supra*; Krieg, 2012, *supra*) and, thus, acts independently of the adaptive immune system.

However, today, none of the tested CpG nucleic acids have been approved for clinical use. While the precise reasons remain unknown, one can speculate that it may be due to a potential toxicity of CpG nucleic acids at the therapeutic doses tested, as suggested by previous studies (Behrens et al., 2011, J. Clin. Invest., 121, 2264-2277), or to the current lack of knowledge about the mechanisms of action of CpG nucleic acids.

In view of the above, there remains a need in the art to provide further and/or improved methods for treating subjects with inflammatory respiratory diseases, in particular chronic respiratory diseases, such as asthma or chronic obstructive pulmonary disease.

### SUMMARY OF THE INVENTION

The present inventors set out to unravel the mechanism behind the contribution of CpG nucleic acid to microbe-induced asthma resistance. Through extensive experiments, as illustrated in the example section herein, the present inventors show in mice that CpG nucleic acid has the unique ability to expand lung interstitial regulatory macrophages (IM) from CCR2-independent monocytes residing in the lung or mobilized from the spleen. Furthermore, CpG nucleic acid-induced IM produced much higher levels of interleukin (IL)-10 than their steady-state counterparts and recapitulated the protective effects of CpG nucleic acid when adoptively transferred to mice before allergen sensitization or challenge by IL-10 dependent mechanisms. As corroborated by the experimental section, which illustrates certain representative embodiments of the invention, the inventors have realised a method for *in vitro* differentiation of monocytes to regulatory macrophages, and the use of the regulatory macrophages in methods of treating inflammatory respiratory diseases, in particular chronic respiratory diseases, such as asthma or COPD, in a subject.

Hence, a first aspect of the present invention relates to a method for *in vitro* or *ex vivo* differentiation of monocytes to regulatory macrophages, the method comprising culturing the monocytes in a medium in the presence of an immunostimulatory nucleic acid.

The methods illustrating the principles of the present invention advantageously allow obtaining hypersuppressive regulatory macrophages using standard culture media and commercially available immunostimulatory nucleic acids such as CpG nucleic acids. Also, the present methods allow obtaining the regulatory macrophages starting from (CCR2-dependent) classical blood monocytes which may be obtained easily from patients. Advantageously, the hypersuppressive regulatory macrophages are useful for the treatment of inflammatory respiratory diseases, in particular chronic respiratory diseases, such as asthma or COPD, thereby overcoming the need for treatment of patients with synthetic CpG nucleic acid, and hence avoiding the risks inherent to CpG nucleic acid injection *in vivo.* Furthermore, the present methods allow obtaining regulatory macrophages that are long-lived and hence advantageously decrease the frequency of administration to patients.

A further aspect relates to regulatory macrophages obtainable or obtained by a method as defined herein.

Yet a further aspect relates to the regulatory macrophages as taught herein for use in treating an inflammatory respiratory disease, in particular a chronic respiratory disease, such as asthma or COPD.

A related aspect provides a method of treating an inflammatory respiratory disease, in particular a chronic respiratory disease, such as asthma or COPD, in a subject in need of such treatment comprising administering to said subject a therapeutically effective amount of the regulatory macrophages as taught herein.

A related aspect provides the use of the regulatory macrophages as taught herein for the manufacture of a medicament for the treatment of an inflammatory respiratory disease, in particular a chronic respiratory disease, such as asthma or COPD.

The ensuing statements provide additional illustration of certain aspects and embodiments that have been disclosed in accordance with the present invention:
1. A method for *in vitro* differentiation of monocytes to regulatory macrophages, comprising culturing the monocytes in a medium in the presence of an immunostimulatory nucleic acid.
2. The method according to statement 1, comprising:
   (a) isolating monocytes from a biological sample of a subject, thereby obtaining monocytes or a cell population comprising monocytes;
   (b) adding the monocytes or the cell population comprising monocytes to a medium comprising an immunostimulatory nucleic acid, thereby obtaining a monocyte-medium mixture;
   (c) culturing the monocyte-medium mixture, thereby obtaining regulatory macrophages or a cell population comprising regulatory macrophages.
3. The method according to statement 1 or 2, wherein the monocytes are isolated from whole blood or bone marrow.
4. The method according to any one of statements 1 to 3, wherein the monocytes are circulating monocytes.
5. The method according to any one of statements 1 to 4, wherein the monocytes are human monocytes.
6. The method according to any one of statements 1 to 5, wherein the monocytes are classical monocytes, such as wherein the monocytes are CD14⁺⁺CD16⁻ cells.
7. The method according to any one of statements 1 to 6, wherein the concentration of monocytes in the medium is between about 1 x 10² and about 1 x 10⁹ cells per ml of the medium, such as wherein the concentration of monocytes in the medium is between about 1 x 10³ and about 1 x 10⁸, or between about 1 x 10⁴ and about 1 x 10⁷ cells per ml of the medium, or between about 1 x 10⁴ and about 1 x 10⁶ cells per ml of the medium, preferably wherein the concentration of monocytes in the medium is between about 5 x 10⁴ and about 2 x 10⁵ cells per ml of the medium.
8. The method according to any one of statements 1 to 7, wherein the immunostimulatory nucleic acid is a CpG nucleic acid.
9. The method according to any one of statements 1 to 8, wherein the immunostimulatory nucleic acid is a nucleic acid consisting of from 6 to 100 nucleotides, such as wherein the immunostimulatory nucleic acid is a nucleic acid consisting of from 6 to 50, from 6 to 40, from 6 to 30, from 8 to 50, from 8 to 40, from 8 to 30, from 10 to 50, from 10 to 40, from 10 to 30, from 15 to 30, or from 18 to 28 nucleotides.
10. The method according to any one of statements 1 to 9, wherein the concentration of the immunostimulatory nucleic acid in the medium is from 0.01 to 200 µg per ml of the medium, such as wherein the concentration of the immunostimulatory nucleic acid in the medium is from 0.025 to 175 µg/ml, from 0.050 to 150 µg/ml, from 0.075 to 125 µg/ml, from 0.10 to 100 µg/ml, from 0.25 to 75 µg/ml, from 0.50 to 50 µg/ml, or from 0.75 to 25 µg/ml of the medium, preferably wherein the concentration of the immunostimulatory nucleic acid in the medium is from 0.1 to 20 µg per ml of the medium.
11. The method according to any one of statements 1 to 10, wherein the monocytes or the cell population comprising monocytes are cultured in a medium in the presence of an immunostimulatory nucleic acid for 24 hours (1 day) to 240 hours (10 days), such as for 24 hours (1 day) to 192 hours (8 days), for 24 hours (1 day) to 144 hours (6 days), for 24 hours (1 day) to 96 hours (4 days), for 24 hours (1 day) to 72 hours (3 days), or for 48 hours (2 days) to 144 hours (6 days), for 48 hours (2 days) to 120 hours (5 days), for 48 hours (2 days) to 96 hours (4 days), or for 48 hours (2 days) to 72 hours (3 days).
12. The method according to any one of statements 1 to 11, wherein the method further comprises culturing the monocytes or the cell population comprising monocytes in the presence of macrophage colony-stimulating factor (M-CSF).
13. The method according to any one of statements 1 to 12, wherein the method further comprises culturing the regulatory macrophages or cell population comprising regulatory macrophages in the presence of one or more antigens, preferably for 1 hour to 24 hours prior to collection of the cells.
14. Regulatory macrophages obtainable or obtained by a method as defined in any one of statements 1 to 13.
15. The regulatory macrophages according to statement 14, for use in treating an inflammatory respiratory disease.
16. The regulatory macrophages for use according to statement 15, wherein the inflammatory respiratory disease is selected from the group consisting of asthma, chronic obstructive pulmonary disease (COPD), chronic bronchitis, acute respiratory distress syndrome (ARDS), pneumonia, respiratory distress of prematurity, cystic fibrosis, pulmonary fibrosis, and pulmonary sarcoidosis.
17. The regulatory macrophages for use according to any one of statement 15 or 16, wherein the regulatory macrophages are to be administered to an autologous subject.
18. The regulatory macrophages for use according to any one of statements 15 to 17, wherein the regulatory macrophages are to be administered by intratracheal, intrabroncheal, or subcutaneous administration.
19. A pharmaceutical composition comprising the regulatory macrophages as defined in statement 14.

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

### BRIEF DESCRIPTION OF THE FIGURES

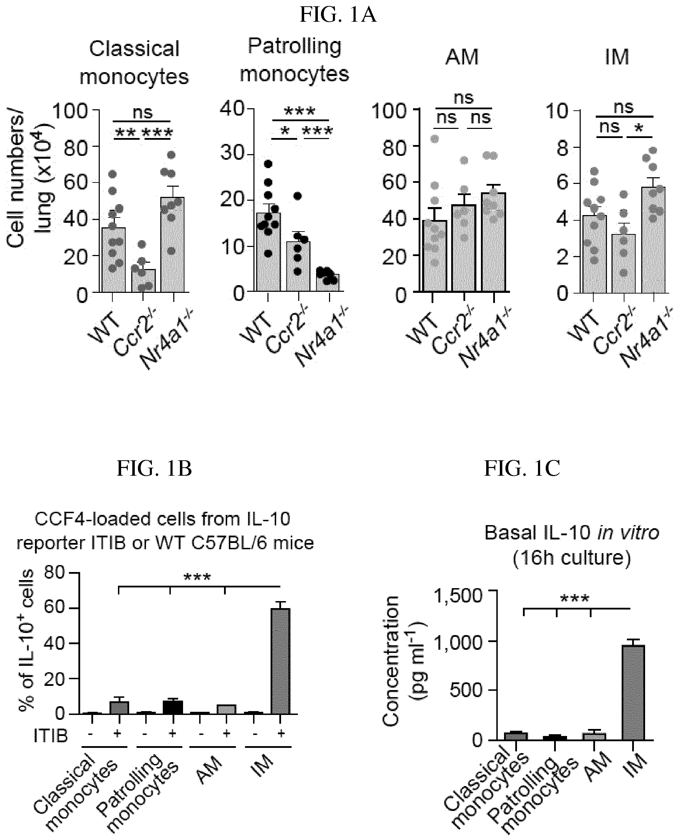

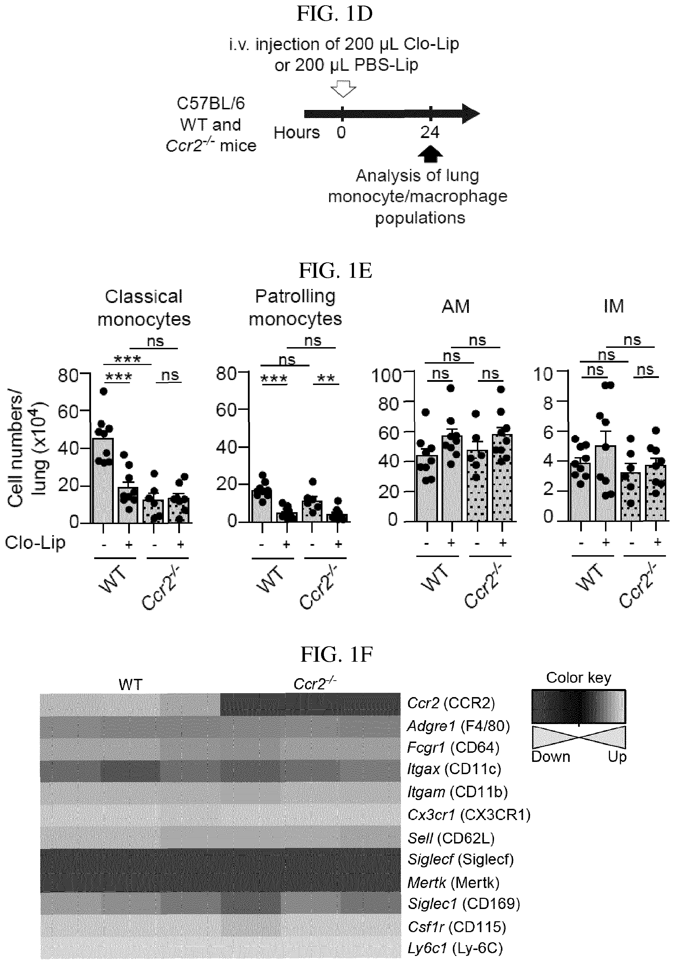

**Figure 1** illustrates the identification of monocyte and macrophage populations in the mouse lung. **Figure 1A** represents graphs illustrating the absolute numbers of the indicated populations (classical monocytes, patrolling monocytes, alveolar macrophages (AM) or interstitial macrophages (IM)) in the lungs of WT, *Ccr2*^{*-*/*-*} and *Nr4a1*^{*-*/*-*} mice. **Figure 1B** represents a graph illustrating the percentage (%) of IL-10⁺ cells within the cell populations as assessed by detection of 450 nm florescence (blue product) in CCF4 substrate-loaded cells isolated from IL-10-β-lactamase reporter ITIB or WT mice. **Figure 1C** represents a graph illustrating the basal IL-10 concentration in culture supernatants of primary cell culture. **Figure ID** represents a schematic overview illustrating the experimental outline for the data in **Figure IE**. **Figure IE** represents graphs illustrating the absolute numbers of the indicated populations in the lungs of PBS-Lip or Clo-Lip i.v.-treated WT and Ccr2^{-/-} mice. **Figure 1F** represents a heatmap showing relative expression of differentially expressed genes (DE) genes of cell surface markers in WT and *Ccr2*^{*-*/*-*} mice. **Figures 1A** **and** **1E****:** Data show mean + SEM and are pooled from 3 independent experiments (n=6-10/group). **Figures 1B and 1C****:** Data show mean + SEM and are representative of 1 of 3 independent experiments (n=3-4/group). *P* values were calculated using a one-way **(****Figure 1A, 1B, and 1C****)** or two-way **(****Figure 1E**) ANOVA followed by Tukey's post hoc tests.

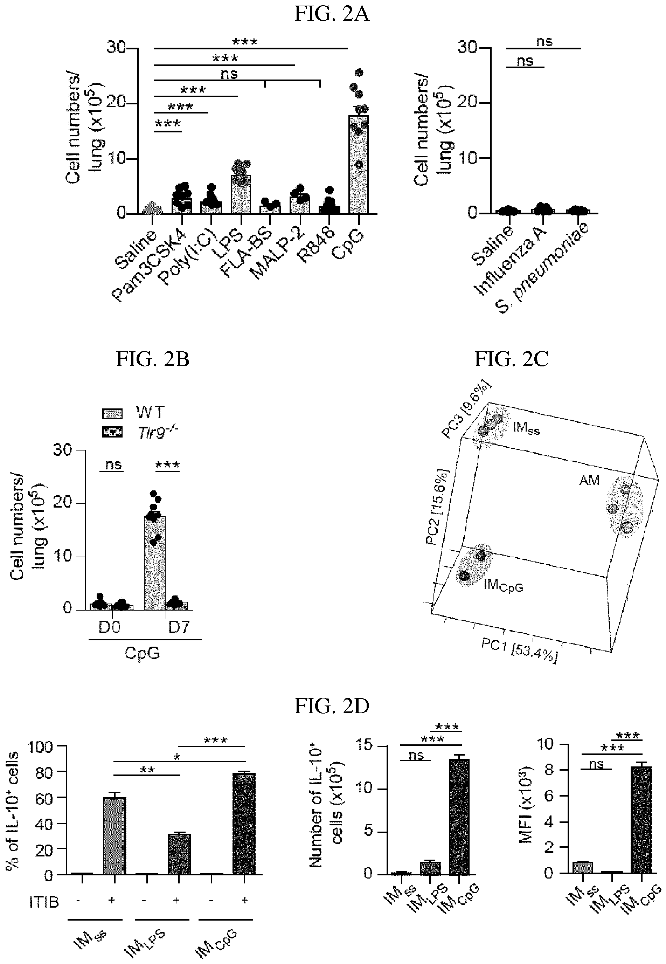

**Figure 2** illustrates the TLR9-dependent expansion of IL-10 producing IM upon intranasal CpG treatment. **Figure 2A** represents graphs illustrating the absolute numbers of IM in the lungs of WT mice 7 days following i.n. stimulation with the indicated TLR ligands (left panel) or i.n. infection with influenza A or S. pneumoniae (right panel). **Figure 2B** represents a graph illustrating the absolute numbers of IM in the lungs of Tlr9^{-/-} and control (WT) mice before and 7 days following i.n. injection of CpG. **Figure 2C** represents a schematic overview of the Principal Component Analysis (PCA) comparing IMₛₛ, IM_{CpG} and AM. Percentages indicate the % of variability explained by each component. **Figure 2D** represents graphs illustrating the percentage (%) (left), numbers (middle) and MFI (right) of IL-10⁺ cells within the cell populations using ITIB mice. **Figures 2A and 2B****:** Data show mean + SEM and are pooled from 3 independent experiments (n=6-12/group). **Figure 2D****:** Data show mean + SEM and are representative of 1 of 2 independent experiments (n=3-4/group). *P* values were calculated using a one-way **(****Figure 2A and 2D****)** or two-way **(****Figure 2B****)** ANOVA followed by Tukey's post hoc tests. D: day; MFI: geometric mean fluorescence intensity.

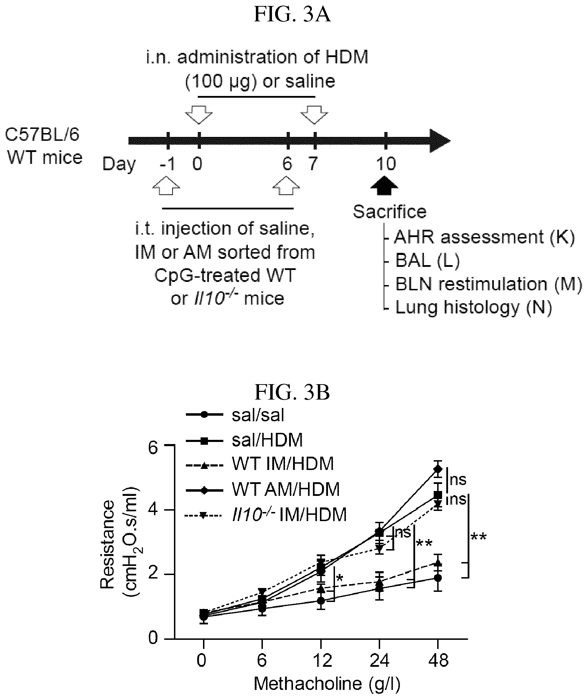

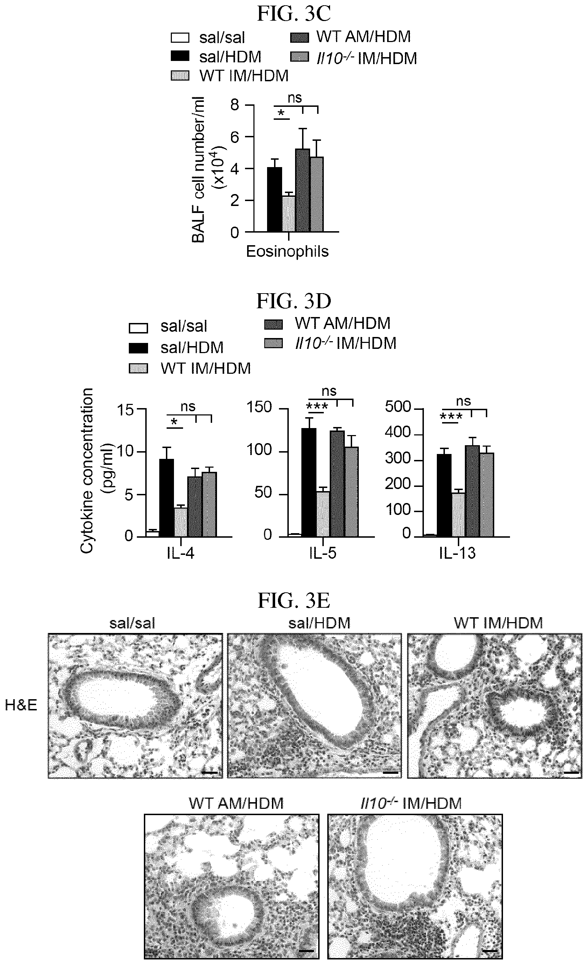

**Figure 3** illustrates the contribution of IM_{CpG} to the prevention of experimental asthma. **Figure 3A** represents a schematic overview illustrating the experimental outline for the data in **Figures 3B** to **3E**, in which saline (control), interstitial macrophages (IM) or alveolar macrophages (AM) sorted from lungs of CpG-treated WT or *IL10*^{*-*/*-*} mice were transferred to C57BL/6 WT mice before house dust mite (HDM) exposure. AHR: airway hyperreactivity; BAL: broncho alveolar lavage; BLN: bronchial lymph node; i.n.: intranasal; i.t.: intratracheal. **Figure 3B** represents a graph illustrating dynamic airway resistance upon methacholine inhalation. **Figure 3C** represents a graph illustrating broncho-alveolar lavage fluid (BALF) eosinophil counts. **Figure 3D** represent graphs illustrating IL-4, IL-5, or IL-13 cytokine concentrations in culture supernatants of bronchial lymph node (BLN) cells restimulated with HDM. **Figure 3E** represents photographs illustrating hematoxylin and eosin (H&E) staining of lung sections. Scale bar: 32 µm. **Figures 3B****-D:** Data show mean + SEM and are pooled from ≥3 independent experiments (n=6-16/group). *P* values were calculated using one-way ANOVA followed by Tukey's post hoc tests. **Figure 1E****:** Data are representative of 1 of ≥6 mice from ≥2 independent experiments.

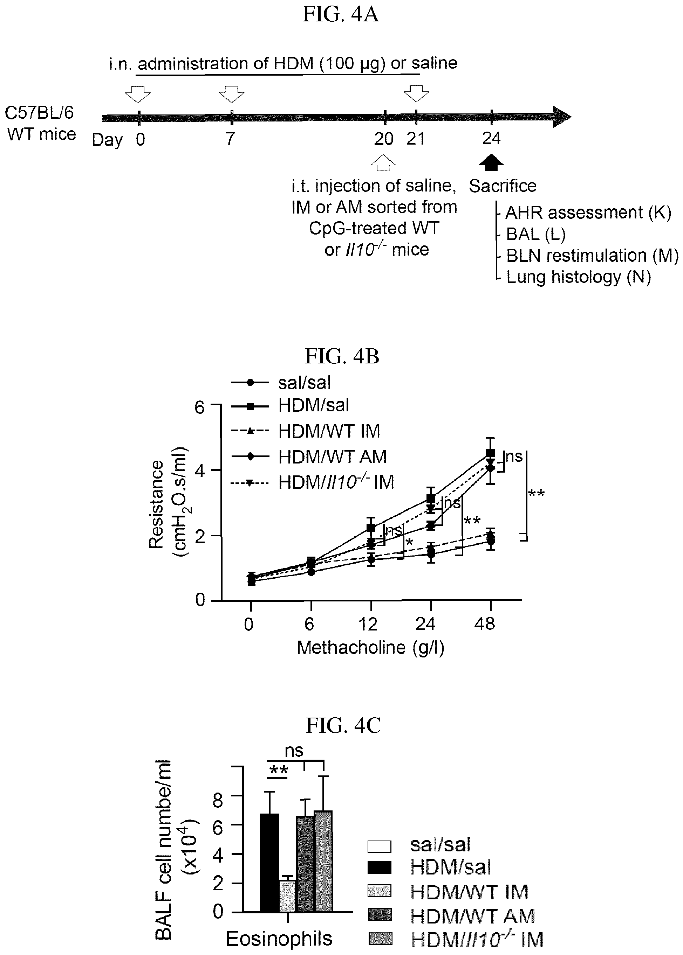

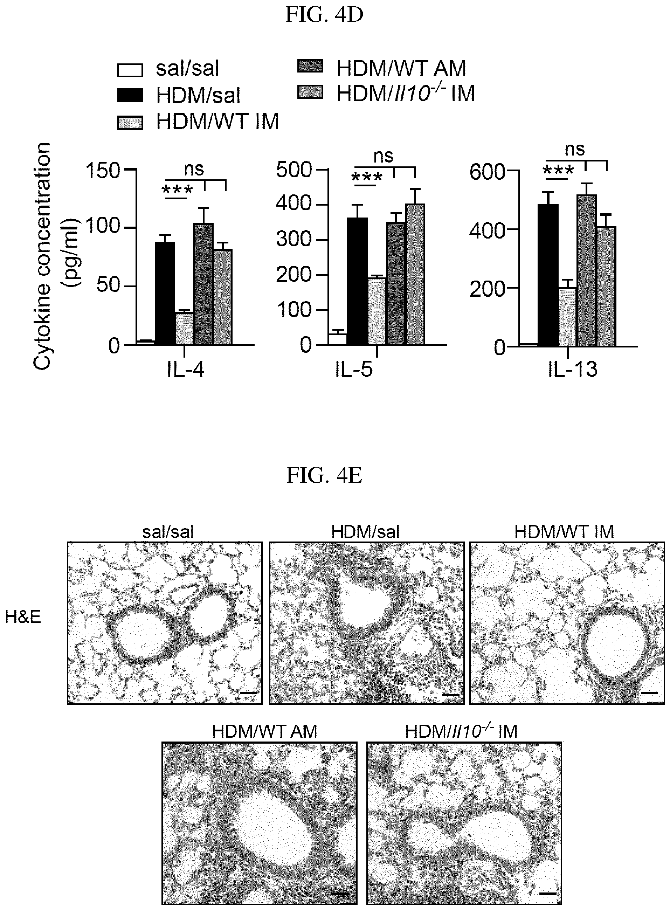

**Figure 4** illustrates the contribution of IM_{CpG} to the treatment of experimental asthma. **Figure 4A** represents a schematic overview illustrating the experimental outline for the data in **Figures 4B** to **4E**, in which saline (control), interstitial macrophages (IM) or alveolar macrophages (AM) sorted from lungs of CpG-treated WT or *IL10*^{*-*/*-*} mice were transferred to HDM-sensitized C57BL/6 WT mice. AHR: airway hyperreactivity; BAL: broncho alveolar lavage; BLN: bronchial lymph node; i.n.: intranasal; i.t.: intratracheal. **Figure 4B** represents a graph illustrating dynamic airway resistance upon methacholine inhalation. **Figure 4C** represents a graph illustrating broncho-alveolar lavage fluid (BALF) eosinophil counts. **Figure 4D** Cytokine concentrations in culture supernatants of BLN cells restimulated with HDM. **Figure 4E** represents photographs illustrating H&E staining of lung sections. Scale bar: 32 µm. **Figures 4B-4D****:** Data show mean + SEM and are pooled from 3 independent experiments (n=6-16/group). *P* values were calculated using one-way ANOVA followed by Tukey's post hoc tests. **Figure 4E****:** Data are representative of 1 of ≥6 mice from ≥2 independent experiments.

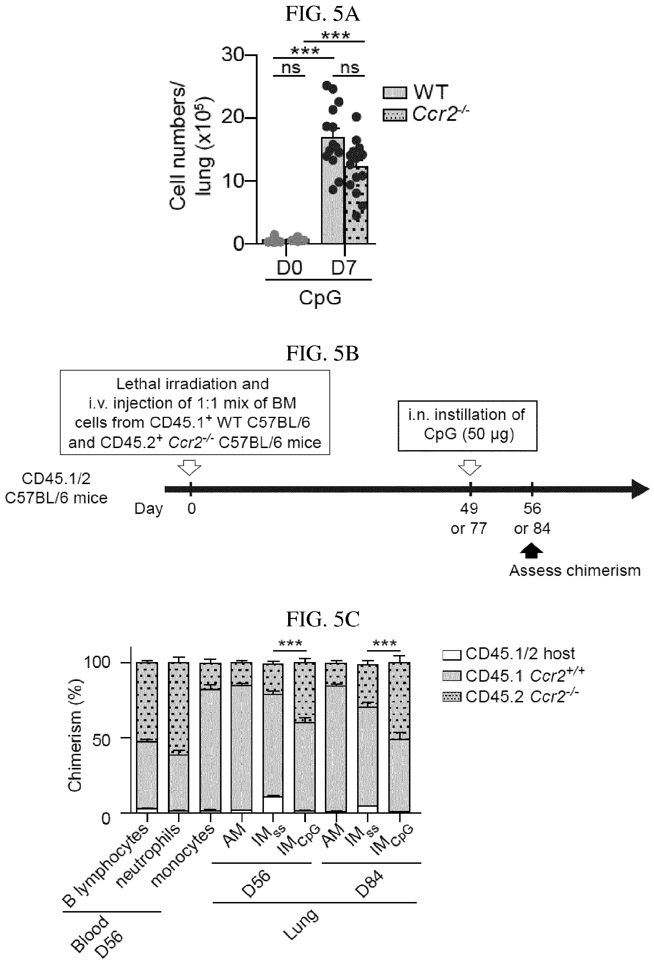

**Figure 5** illustrates the level of dependency of IM_{CpG} on CCR2. **Figure 5A** represents a graph illustrating the absolute numbers of IM in the lungs of WT and *Ccr2*^{*-*/*-*} mice before and 7 days after i.n. CpG treatment (50 µg). **Figure 5B** represents a schematic overview illustrating the experimental outline for experiments using bone marrow (BM) mixed competitive chimeras shown in **Figure 5C**. i.v.: intravenous; i.n.: intranasal. **Figure 5C** represent a graph illustrating the percentage (%) of BM chimerism and radio-resistance of the indicated populations. **Figure 5C****:** The % of chimerism and the % of CCR2 independency of blood B lymphocytes, neutrophils (i.e., 50% chimerism, 100% CCR2 independency) and monocytes (i.e. ±10% chimerism, ±20% CCR2 independency) were used as controls. **Figures 5A and 5C****:** Data show mean + SEM and are pooled from 3 independent experiments (n=12-15/group). *P* values were calculated using a two way **(****Figure 5A**) or a one-way **(****Figure 5C**) ANOVA followed by Tukey's post hoc tests. D: day.

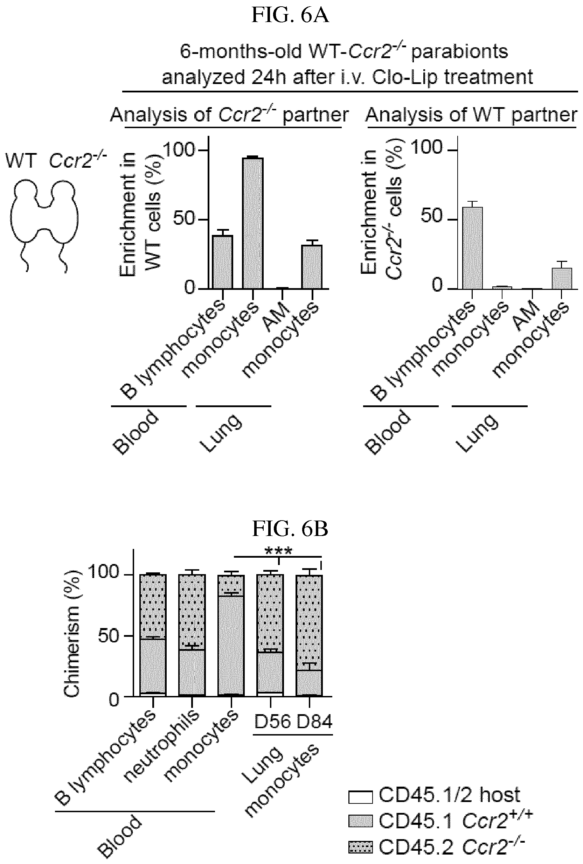

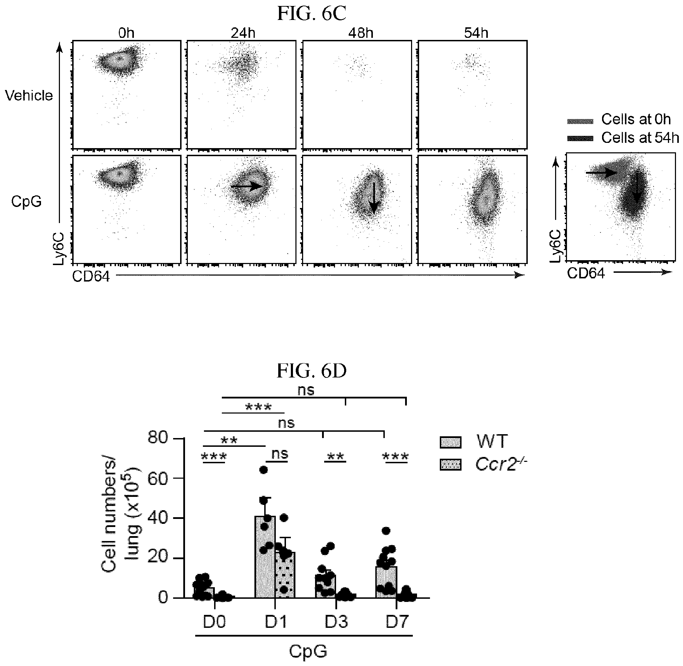

**Figure 6** illustrates CpG-induced differentiation of IM from TLR9-responsive CCR2-independent lung monocytes. **Figure 6A** represents graphs illustrating the analysis of 6-month-old *WT-Ccr2*^{*-*/*-*} parabionts 24 hours after i.v. Clo-Lip treatment. The percentage (%) of enrichment in partner cells within each host is shown. **Figure 6B** represents a graph illustrating the percentage (%) of BM chimerism and radio-resistance of the indicated populations. BM mixed competitive chimeras were analyzed at D56 or D84, 24 hours after i.v. injection of Clo-Lip. **Figure 6C** (left panel) represents dot plots illustrating the waterfall of lung monocytes sorted from *Ccr2*^{*-*/*-*} mice and cultured *ex vivo* with vehicle or CpG; (right panel) Superposition of dot plots of living lung monocytes before and 54 hours after stimulation with CpG. **Figure 6D** represents a graph illustrating absolute numbers of Ly-6C^{hi} lung monocytes in WT and *Ccr2*^{*-*/*-*} mice before and 1, 3 and 7 days after i.n. CpG instillation. **Figure 6C****:** Data are representative of one of ≥6 samples coming from 3 independent experiments. **Figure 6A****:** Bar graphs (mean + SEM) showing summary data pooled from two pairs of parabionts. **Figures 6B** **and** **6D****:** Data show mean + SEM and are pooled from 3 independent experiments (n=6-15/group). *P* values were calculated using a one-way **(****Figure 6B****)** or a two-way **(****Figure 6C****)** ANOVA followed by Tukey's post hoc tests. D: day.

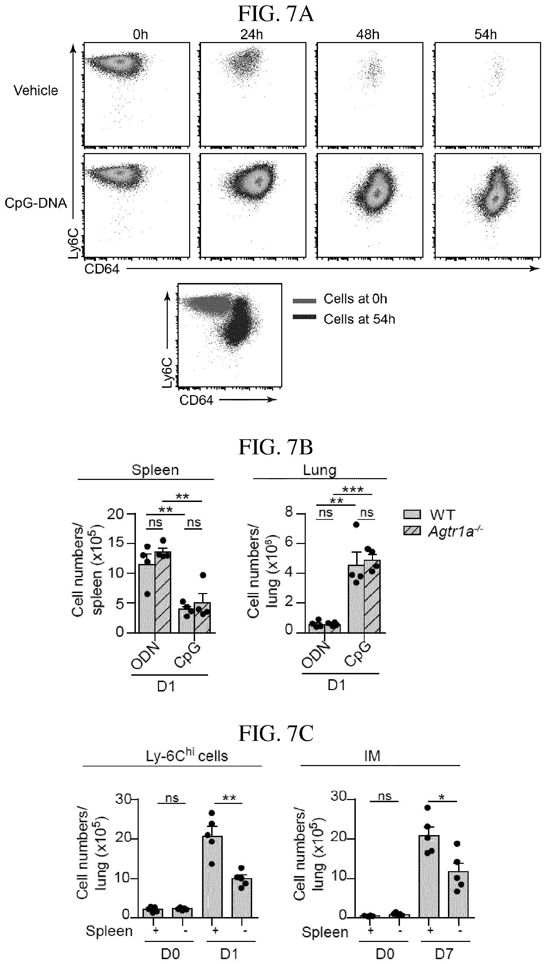

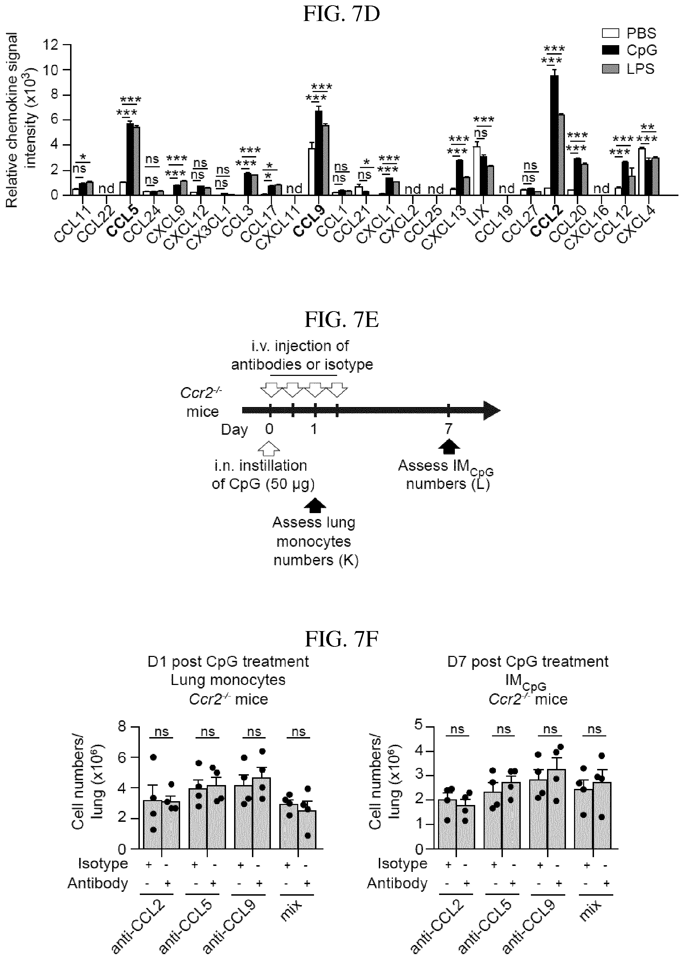

**Figure 7** illustrates mobilization of CCR2-independent spleen reservoir monocytes to the lung and differentiation into IM_{CpG.} **Figure 7A** represents dot plots illustrating the waterfall of splenic monocytes sorted from *Ccr2*^{*-*/*-*} mice and stimulated *ex vivo* with vehicle or CpG (upper panel). On the lower panel, superposition of dot plots of living lung monocytes before and 54 hours after stimulation with CpG. **Figure 7B** represents graphs illustrating absolute numbers of Ly-6C^{hi} monocytes in the spleen (left panel) and in the lung (right panel) of WT and *Agtr1a*^{*-*/*-*} mice 1 day after i.n. CpG treatment. **Figure 7C** represent graph illustrating absolute numbers of Ly-6C^{hi} monocytes (left panel) or IM (right panel) in lung of splenectomized and sham-operated *Ccr2*^{*-*/*-*} mice before (DO) and 1 day (left panel) or 7 days (right panel) after i.n. CpG treatment. **Figure 7D** represents a graph illustrating chemokine array performed on whole lung proteins extracted 8 hours after i.n. PBS, LPS or CpG treatment of WT mice. Quantification of chemokine signal intensity. **Figure 7E** represents a schematic overview of the experimental outline for *in vivo* chemokine neutralization experiments shown in **Figure 7F. Figure 7F** represents graphs illustrating the absolute numbers of lung monocytes 1 day (left panel) or IM_{CpG} 7 days (right panel) after i.n. CpG instillation in *Ccr2*^{*-*/*-*} mice which received i.p. injection of the indicated antibodies. **Figure 7A**: Data are representative of one of ≥4 samples coming from 2 independent experiments. **Figures 7B**, **7C****,** **7D, 7F****:** Data show mean + SEM and (7C, 7D) are pooled from 2 or 3 independent experiments (n=4-6/group); (7B, 7F) representative of 1 of 2 independent experiments (n=4/group). *P* values were calculated using a two-way ANOVA followed by Tukey's post hoc tests. D: day.

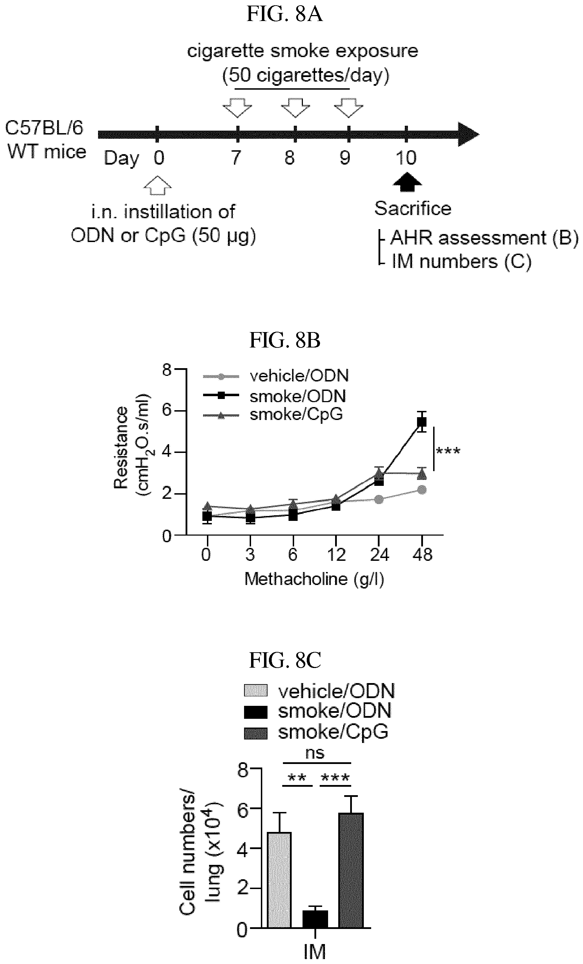

**Figure 8** illustrates a therapeutic and protective role of CpG-induced IM in cigarette smoke-induced lung pathology. **Figure 8A** represent a schematic overview of the experimental outline for data in **Figures 8B-8C. Figure 8B** represents a graph illustrating dynamic airway resistance upon methacholine inhalation. **Figure 8C** represents a graph illustrating absolute numbers of IM in the lungs. AHR: airway hyperreactivity; IM: interstitial macrophages.

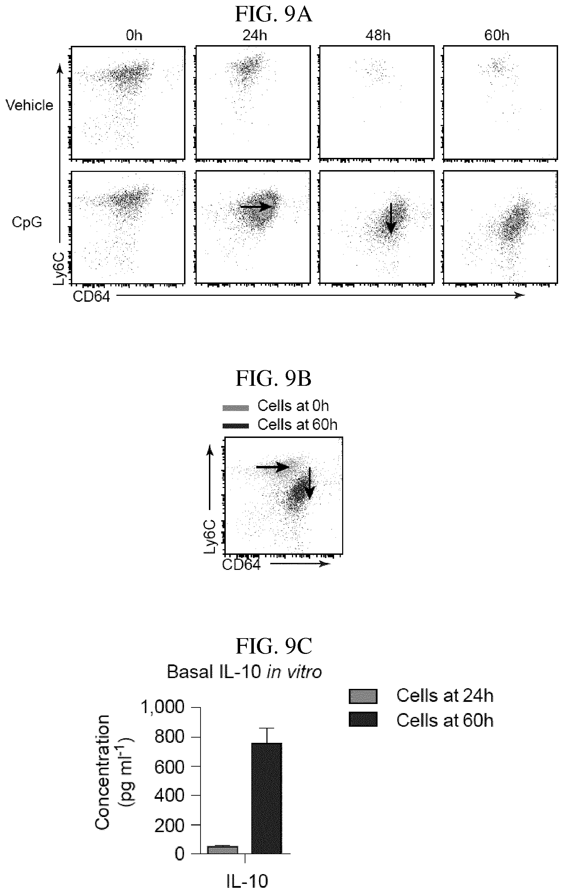

**Figure 9** illustrates a method for *in vitro* differentiation of monocytes to regulatory macrophages according to an embodiment of the invention. **Figure 9A** represents dot plots of living CCR2-dependent classical monocytes (Ly-6C^{hi} monocytes) sorted from C57BL/6 WT mice and cultured *ex vivo* before (0h) and 24, 48 or 60 hours (24h, 48h and 60h) after stimulation with vehicle or CpG. **Figure 9B** represents a superposition of dot plots of living Ly-6C^{hi} monocytes sorted from C57BL/6 WT mice and cultured *ex vivo* before (0h) and 60 hours (60h) after stimulation with CpG. **Figure 9C** represents a graph illustrating basal IL-10 concentration in culture supernatants of FACS-sorted Ly-6C^{hi} monocytes from C57BL/6 WT mice after 24 (24h) and 60-hours (60h) culture.

### DETAILED DESCRIPTION OF THE INVENTION

Before the present method and products of the invention are described, it is to be understood that this invention is not limited to particular methods, components, products or combinations described, as such methods, components, products and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, *e.g.,* any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

The present inventors have found that *in vitro* culturing of blood (classical) monocytes in the presence of an immunostimulatory nucleic acid induces their differentiation to regulatory macrophages exhibiting a hypersuppressive profile.

Hence, the present invention provides a method for *in vitro* differentiation of monocytes to regulatory macrophages, comprising culturing the monocytes in a medium in the presence of an immunostimulatory nucleic acid.

The term "*in vitro*" generally denotes outside, or external to, animal or human body. The term "*ex vivo*" typically refers to tissues or cells removed from an animal or human body and maintained or propagated outside the body, e.g., in a culture vessel. The term "*in vitro*" as used herein should be understood to include *"ex vivo*". The term "*in vivo*" generally denotes inside, on, or internal to, animal or human body.

The present invention provides methods for *in vitro* differentiation of monocytes as defined herein to regulatory macrophages as defined herein.

The phrases "methods for *in vitro* differentiation of monocytes to regulatory macrophages" or "methods for *in vitro* differentiating monocytes to regulatory macrophages" may be used interchangeably.

The term "differentiation" as used herein refers to the sum of the processes whereby unspecialized or less specialized cells (e.g., monocytes such as blood monocytes) attain a more complex, more specialized or adult form and function (e.g., regulatory macrophages).

The method advantageously allows differentiating blood monocytes - which can be easily obtained from whole blood, for example of a human subject - to regulatory macrophages, which can then be transferred back to the human subject, for instance by intratracheal or intrabronchial instillation, for the treatment of an inflammatory respiratory disease, in particular a chronic respiratory disease, such as asthma or COPD.

### Monocytes

In certain embodiments, the invention provides a method for *in vitro* differentiation of monocytes to regulatory macrophages.

The term "monocyte" is well-known in the art and generally refers to a type of white blood cell or leukocyte.

In certain embodiments, the monocytes as taught herein may be isolated from whole blood, bone marrow, spleen and/or lung. In certain embodiments, the monocytes as taught herein may be isolated from whole blood and/or bone marrow.

In certain embodiments of the methods as taught herein, the monocytes as taught herein may be isolated from whole blood. Monocytes isolated from whole blood are advantageous in that they can be easily obtained from the blood of a subject (e.g. the subject to be treated). Blood of a subject can be obtained in a straightforward manner such as by apheresis (in which blood of a subject is passed through an apparatus that separates out one particular constituent, e.g. monocytes, and returns the remainder to the circulation) or a standard blood donation (in which blood is drawn from a subject).

The monocytes can be isolated from the blood by any adequate technique known to the skilled person for whole blood monocytes isolation. Suitable techniques for the isolation of monocytes from whole blood include apheresis, optionally followed by an enrichment of monocytes by magnetic-activated cell sorting (MACS) or by flow cytometry such as fluorescence-activated cell sorting (FACS); flow cytometry, such as FACS; MACS; density gradient centrifugation, e.g. density gradient centrifugation followed by MACS or by flow cytometry such as FACS; or a combination thereof. Preferably, monocytes are isolated form whole blood by apheresis, optionally followed by an enrichment of monocytes by MACS or by flow cytometry such as FACS.

Suitable techniques for isolation of monocytes from the bone marrow, spleen and/or lung include flow cytometry, such as fluorescence-activated cell sorting (FACS); magnetic-activated cell sorting (MACS); density gradient centrifugation, e.g. density gradient centrifugation followed by MACS or by flow cytometry such as FACS; or a combination thereof.

In certain embodiments of the methods as taught herein, the monocytes may be circulating monocytes. Circulating monocytes can be easily obtained from the blood of a subject (e.g. the subject to be treated), whereby the blood can be obtained in a straightforward manner by a standard blood donation.

The term "circulating monocytes" refers to monocytes that are transported around the body (i.e. circulate) through the circulatory or vascular system.

In certain embodiments of the methods as taught herein, the monocytes as taught herein and/or cells derived thereof including the regulatory macrophages as taught herein, may be animal cells, preferably warm-blooded animal cells, more preferably mammalian cells, such as human cells or non-human mammalian cells, and most preferably human cells.

In certain preferred embodiments, the monocytes are human monocytes. In embodiments, the monocytes may comprise one or more of classical monocytes, non-classical monocytes, and intermediate monocytes. The human classical monocytes, non-classical monocytes, and intermediate monocytes are defined as known in the art (Ginhoux and Jung, 2014, Nat. Rev. Immunol., 14, 392-404).

In embodiments, the monocytes may comprise or consists of classical monocytes characterized by high level expression of CD14 and no expression of CD16 (e.g. CD14⁺⁺CD16⁻ cells). In certain embodiments, the classical monocytes may be further characterized by high level expression of CC-chemokine receptor 2 (CCR2) (e.g. CCR2^{hi} cells). In certain embodiments, the classical monocytes may be further characterized by low level expression of CX₃C-chemokine receptor 1 (CX₃CR1) (e.g. CX₃CR1^{low} cells). In certain embodiments, the classical monocytes may be further characterized by high level expression of CCR2 and low level expression of CX₃CR1 (e.g. CCR2^{hi}CX₃CR1^{low} cells). In embodiments, the monocytes may comprise or consist of non-classical monocytes characterized by low level expression of CD 14 and high level expression of CD16 (e.g. CD14⁺CD16⁺⁺ cells). In certain embodiments, the non-classical monocytes may be further characterized by high level expression of CX₃CR1 (e.g. CX₃CR1^{hi} cells). In certain embodiments, the non-classical monocytes may be further characterized by low level expression of CCR2 (e.g. CCR2^{low} cells). In certain embodiments, the non-classical monocytes may be further characterized by high level expression of CX₃CR1 and low level expression of CCR2 (e.g. CX₃CR1^{hi}CCR2^{low} cells). In embodiments, the monocytes may comprise or consist of intermediate monocytes characterized by high level expression of CD 14 and low level expression of CD16 (e.g. CD14⁺⁺CD16⁺ cells). In certain embodiments, the intermediate monocytes may be further characterized by high level expression of CX₃CR1 (e.g. CX₃CR1^{hi} cells). In certain embodiments, the intermediate monocytes may be further characterized by low level expression of CCR2 (e.g. CCR2^{low} cells). In certain embodiments, the intermediate monocytes may be further characterized by high level expression of CX₃CR1and low level expression of CCR2 (e.g. CX₃CR1^{hi}CCR2^{low} cells).

In certain embodiments, the monocytes may be classical monocytes. In certain embodiments, the monocytes may be classical monocytes characterized by high level expression of CD 14 and no expression of CD16 (e.g. CD14⁺⁺CD16⁻ cells). In certain embodiments, the monocytes may be human classical monocytes. In certain embodiments, the monocytes may be human classical monocytes characterized by high level expression of CD 14 and no expression of CD16. In certain embodiments, the monocytes may be CD14⁺⁺CD16⁻ cells. In certain embodiments, the monocytes may be CD14⁺⁺CD16⁻ CCR2^{hi}CX₃CR1^{low} cells.

In certain embodiments, the monocytes may be CCR2-dependent classical monocytes. Such cells may be easily isolated from whole blood of a subject (e.g. the subject to be treated). Typically, CCR2-dependent classical monocytes originate from the bone marrow. In certain embodiments, the monocytes may be CCR2-dependent classical monocytes originating from bone-marrow.

In certain embodiments, the monocytes may be mouse monocytes. In embodiments, the monocytes may comprise one or more of Ly6C^{hi} monocytes and Ly6C^{low} monocytes. The mouse Ly6C^{hi} monocytes and Ly6C^{low} monocytes are defined as known in the art (Ginhoux and Jung, 2014, Nat. Rev. Immunol., 14, 392-404).

In embodiments, the monocytes may comprise or consists of Ly6C^{hi} monocytes characterized by high level expression of Ly6C or GR1, expression of CD11b and expression of CD115 (e.g. CD11b⁺CD115⁺Ly6C^{hi} cells). In certain embodiments, the Ly6C^{hi} monocytes may be further characterized by high level expression of CC-chemokine receptor 2 (CCR2). In certain embodiments, the Ly6C^{hi} monocytes may be further characterized by low level expression of CX₃C-chemokine receptor 1 (CX₃CR1). In certain embodiments, the Ly6C^{hi} monocytes may be further characterized by high level expression of CCR2 and low level expression of CX₃CR1 (e.g. Ly6C^{hi}CCR2^{hi}CX₃CR1^{low} cells). In embodiments, the monocytes may comprise or consists of Ly6C^{low} monocytes characterized by low level expression of Ly6C, expression of CD11b and expression of CD115 (e.g. CD11b⁺CD115⁺Ly6C^{low} cells). In certain embodiments, the Ly6C^{low} monocytes may be further characterized by high level expression of CX₃CR1. In certain embodiments, the Ly6C^{low} monocytes may be further characterized by low level expression of CCR2. In certain embodiments, the Ly6C^{low} monocytes may be further characterized by high level expression of CX₃CR1 and low level expression of CCR2 (e.g. Ly6C^{low}CX₃CR1^{hi}CCR2^{low} cells).

In embodiments, the monocytes may be mouse Ly6C^{hi} monocytes. In embodiments, the monocytes may be mouse Ly6C^{hi} monocytes characterized by high level expression of Ly6C, expression of CD11b and expression of CD115 (e.g. the monocytes may be CD11b⁺CD115⁺Ly6C^{hi} cells). In embodiments, the monocytes may be mouse Ly6C^{hi}CCR2^{hi}CX₃CR1^{low} cells. In certain embodiments, the monocytes may be CD11b⁺CD115⁺Ly6C^{hi} CCR2^{hi}CX₃CR1^{low} cells.

In certain embodiments, the mouse monocytes may display the following characteristics: expression of CD45, and expression of Ly6C. In certain embodiments, the mouse monocytes may be characterized by expression of CD115.

In certain embodiments, the mouse monocytes may be CD45⁺Ly-6C⁺CD64⁻ cells. In certain embodiments, the mouse monocytes may be CD45⁺CD115⁺Ly-6C⁺CD64⁻ cells.

Wherein a cell is said to be positive for (or to express or comprise expression of) a particular (cell surface) marker, this means that a skilled person will conclude the presence or evidence of a distinct signal, e.g., antibody-detectable or detection by reverse transcription polymerase chain reaction, for that marker when carrying out the appropriate measurement, compared to suitable controls. Where the method allows for quantitative assessment of the marker, positive cells may on average generate a signal that is significantly different from the control, e.g., but without limitation, at least 1.5-fold higher than such signal generated by control cells, e.g., at least 2-fold, at least 4-fold, at least 10-fold, at least 20-fold, at least 30-fold, at least 40-fold, at least 50-fold higher or even higher.

Where the method allows for quantitative assessment of the marker, cells characterized by high level expression of a particular (cell surface) marker may on average generate a signal that is significantly different from the control, e.g., but without limitation, at least 3-fold higher than such signal generated by control cells, e.g., at least 4-fold, at least 5-fold, at least 10-fold, at least 20-fold, at least 30-fold, at least 40-fold, at least 50-fold higher or even higher.

Where the method allows for quantitative assessment of the marker, cells characterized by low level expression of a particular (cell surface) marker may on average generate a signal that is significantly different from the control, e.g., but without limitation, 1.5-fold to 2.9-fold higher than such signal generated by control cells, e.g., about 1.5-fold, about 1.6-fold, about 1.7-fold, about 1.8-fold, about 1.9-fold, about 2-fold, about 2.1-fold, about 2.2-fold, about 2.3-fold, about 2.4-fold, about 2.5-fold, about 2.6-fold, about 2.7-fold, about 2.8-fold, or about 2.9-fold higher than such signal generated by control cells.

The expression of the above cell-specific markers can be detected using any suitable immunological technique known in the art, such as immuno-cytochemistry or affinity adsorption, Western blot analysis, flow cytometry (e.g., FACS), ELISA, etc., or by any suitable biochemical assay of enzyme activity (e.g., for ALP), or by any suitable technique of measuring the quantity of the marker mRNA, e.g., Northern blot, semi-quantitative or quantitative RT-PCR, etc. Sequence data for markers listed in this disclosure are known and can be obtained from public databases such as GenBank (http://www.ncbi.nlm.nih.gov/).

Wherein a detection method allows to evaluate the expression of a marker on a single cell level (e.g., flow cytometry), and wherein the cells are said to comprise expression of the marker, a sizeable fraction of the tested cells may be positive for the marker, e.g., at least about 20%, at least about 40%, preferably at least about 50%, more preferably at least about 60%, even more preferably at least about 70%, still more preferably at least about 80%, yet more preferably at least about 90%, and still more preferably at least about 95% and up to 100% of the tested cells may be positive for the marker.

In certain embodiments, the monocytes as taught herein may be obtained from (e.g. isolated from) a biological sample of a subject.

The term "subject" or "patient" are used interchangeably and refer to animals, preferably warm-blooded animals, more preferably vertebrates, and even more preferably mammals specifically including humans and non-human mammals, that have been the object of treatment, observation or experiment. The term "mammal" includes any animal classified as such, including, but not limited to, humans, domestic and farm animals, zoo animals, sport animals, pet animals, companion animals and experimental animals, such as, for example, mice, rats, hamsters, rabbits, dogs, cats, guinea pigs, cattle, cows, sheep, horses, pigs and primates, e.g., monkeys and apes. Particularly preferred are human subjects, including both genders and all age categories thereof.

Non-human animal subjects may also include prenatal forms of animals, such as, e.g., embryos or foetuses. Human subjects may also include foetuses, but by preference not embryos.

The term "biological sample" or "sample" as used herein generally refers to a sample obtained from a biological source, e.g., from an organism, organ, tissue or cell culture, etc. A biological sample of an animal or human subject refers to a sample removed from an animal or human subject and comprising cells thereof. The biological sample of an animal or human subject may comprise one or more tissue types and cells of one or more tissue types. Methods of obtaining biological samples of an animal or human subject are well known in the art, e.g., drawing blood.

In certain embodiments, the biological sample may be whole blood, e.g. obtained by drawing blood; bone marrow, e.g. bone marrow aspirate; spleen, e.g. tissue homogenate obtained by mechanical disruption (e.g. grinding) of spleen tissue; or lung, e.g. cell suspension obtained by lung tissue digestion with enzymes. In preferred embodiments, the sample may be whole blood. Preferably the sample is readily obtainable by minimally invasive methods, allowing the removal or isolation of said sample from the subject, such as by drawing blood. Samples may also include tissue samples, tissue homogenates and the like.

In an embodiment, the monocytes may be obtained from (e.g. isolated from) a healthy subject. In another embodiment, the monocytes may be obtained from (e.g. isolated from) a diseased subject. In certain embodiments, the monocytes may be obtained from (e.g. isolated from) a subject who has an inflammatory respiratory disease, in particular a chronic respiratory disease, for instance asthma or COPD.

The monocytes may be grown as an adherent monolayer on a surface and/or may be in cell suspension (i.e., non-adherent cells).

In certain embodiments, monocytes as taught herein may be adherent, i.e., require a surface for growth, and typically grow as an adherent monolayer on said surface (i.e., adherent cell culture). Adhesion of cells to a surface, such as the surface of a tissue culture plastic vessel, can be readily examined by visual inspection under inverted microscope. In general, a surface or substrate which allows adherence of cells thereto may be any substantially hydrophilic substrate. As known in the art, tissue culture vessels, e.g., culture flasks, well plates, dishes, or the like, may be usually made of a large variety of polymeric materials, suitably surface treated or coated after moulding in order to provide for hydrophilic substrate surfaces

In certain embodiments, monocytes as taught herein may be free-floating cells in a culture medium (suspension culture). The terms "suspension" and "cell suspension" generally refers to a heterogeneous mixture containing cells dispersed in a liquid phase. As the composition is liquid, the cells may in principle be able to, but need not, settle or sediment in the liquid.

The methods as taught herein generally relate to *in vitro* differentiation of living (viable) monocytes. Preferably, the monocytes are functional cells.

The terms "living cells" or "viable cells" as used herein refer to cells that can be qualified as viable by tests known *per se,* and more particularly refer to monocytes that are capable of dividing and proliferating. Where monocytes are said to be living or viable, a sizeable fraction of the tested cells may test as viable, e.g., at least about 20%, at least about 40%, preferably at least about 50%, more preferably at least about 60%, even more preferably at least about 70%, still more preferably at least about 80%, yet more preferably at least about 90%, and still more preferably at least about 95% and up to 100% of the tested cells may test as viable.

Viability of cells may be measured using techniques known in the art. Techniques for determining viability or cell survival are commonly referred to as viability assays. For instance, the viability of cells may be measured using conventional dye exclusion assays, such as Trypan Blue exclusion assay or propidium iodide exclusion assay. In such assays, viable cells exclude the dye and hence remain unstained, while non-viable cells take up the dye and are stained. The cells and their uptake of the dye can be visualised and revealed by a suitable technique (e.g., conventional light microscopy, fluorescence microscopy, flow cytometry), and viable (unstained) and non-viable (stained) cells in the tested sample can be counted. Cell survival can be conveniently expressed as the absolute number of living cells, or as cell viability (i.e., the ratio or proportion (%) of viable cells to total cells [i.e., sum of viable and non-viable cells]).

The term "functional cells" as used herein refers to monocytes that retain their natural cellular properties and are capable of performing their natural cellular functions, such as cell recovery (e.g., the ability of the cells to adhere to a surface), cell identity (e.g., the expression of key cell surface markers), and cell potency (e.g., potency to differentiate into macrophages or dendritic cells). Where monocytes are said to be functional, a sizeable fraction of the tested cells may have retained their natural cellular properties, e.g., at least about 20%, at least about 40%, preferably at least about 50%, more preferably at least about 60%, even more preferably at least about 70%, still more preferably at least about 80%, yet more preferably at least about 90%, and still more preferably at least about 95% and up to 100% of the tested cells.

Functionality of cells may be measured using techniques known in the art. Techniques for determining functionality of cells include assays for measuring known cellular functions, such as cell recovery, cell identity, and cell potency.

For instance, cell recovery can be measured by determining the number or fraction (%) of the monocytes that attach to tissue culture plastic surface after a predetermined time (e.g., 24 hours) in culture. The adherent cells may be visualised by conventional light microscopy. Suitable staining methods, e.g., Romanowsky-type stains, may be used to improve the visualisation of the cells.

For instance, cell identity can be determined by detecting the expression of key cell surface markers characterising the monocytes.

By means of example but without limitation, suitable cell surface markers to evaluate cell identity of monocytes may include CD14 and CD16 (human) or Ly-6C, CD11b, and CD115 (mouse), CD64 and CD45. These cell surface markers can for instance be detected by commercially available monoclonal antibodies, such as fluorochrome-labelled monoclonal antibodies allowing for cell detection by flow cytometry. In particular, CD14 (human) or Ly-6C (mouse) is a monocyte marker, and is typically highly expressed by classical monocytes; CD64 is a macrophage marker, and is typically substantially absent from monocytes.

In certain embodiments, the methods of the present invention may employ monocytes which are autologous to the subject to be treated. The term "autologous", with reference to the monocytes, denotes that the monocytes are obtained from the same subject to be contacted or treated with the regulatory macrophages obtained by the methods as taught herein. The methods of the present invention may employ monocytes which are "allogeneic" to the subject to be treated, i.e., obtained from one or more (pooled) subjects other than the subject to be contacted or treated with the regulatory macrophages obtained by the methods as taught herein. The methods of the present invention may also employ a mixture of autologous and allogeneic monocytes as defined above. Preferably, the methods of the present invention may employ monocytes which are "autologous" to the subject to be treated. Advantageously, autologous monocytes result in regulatory macrophages which are autologous to the subject to be contacted or threated therewith.

In certain embodiments of the methods as taught herein, the concentration of monocytes (preferably of living monocytes, or preferably of living and functional monocytes) in the medium may be between about 1 x 10² and about 1 x 10⁹, or between about 1 x 10³ and about 1 x 10⁸, or between about 1 x 10⁴ and about 1 x 10⁷ cells per ml of the medium. In certain embodiments, the concentration of monocytes in the medium may be between about 1 x 10⁴ and about 1 x 10⁶ cells per ml of the medium. In certain embodiments, the concentration of monocytes in the medium may be between about 5 x 10⁴ and about 1 x 10⁵ cells per ml of the medium, or between about 1 x 10⁵ and about 2 x 10⁵ cells per ml of the medium. In certain embodiments, the concentration of monocytes in the medium may be between about 5 x 10⁴ and about 2 x 10⁵ cells per ml of the medium. In certain embodiments, the concentration of monocytes in the medium may be about 1 x 10⁵ cells per ml of the medium.

### Immunostimulatory nucleic acid

In certain embodiments, the methods as taught herein comprise culturing the monocytes in a medium in the presence of an immunostimulatory nucleic acid, such as a CpG nucleic acid.

By "nucleic acid" is meant oligomers and polymers of any length composed essentially of nucleotides, e.g., deoxyribonucleotides and/or ribonucleotides. Nucleic acids can comprise purine and/or pyrimidine bases and/or other natural (e.g., xanthine, inosine, hypoxanthine), chemically or biochemically modified (e.g., methylated), non-natural, or derivative nucleotide bases. The backbone of nucleic acids can comprise sugars and phosphate groups, as can typically be found in RNA or DNA, and/or one or more modified or substituted sugars and/or one or more modified or substituted phosphate groups. Modifications of phosphate groups or sugars may be introduced to improve stability, resistance to enzymatic degradation, or some other useful property. A "nucleic acid" can be for example double-stranded, partly double stranded, or single-stranded. Where single-stranded, the nucleic acid can be the sense strand or the antisense strand. In addition, nucleic acid can be circular or linear. The term "nucleic acid" as used herein preferably encompasses DNA and RNA, specifically including RNA, genomic RNA, cDNA, DNA, provirus, pre-mRNA and mRNA.

An "immunostimulatory nucleic acid" as used herein refers to any nucleic acid containing an immunostimulatory motif or backbone capable of activating an innate immune response. An immunostimulatory nucleic acid may activate the innate immune system by activating the toll-like receptor 9 (TLR9) (also referred to as cluster of differentiation 289 or CD289). Immunostimulatory motifs include, but are not limited to, CpG motifs, poly-G motifs, and T-rich motifs. Immunostimulatory backbones include, but are not limited to, phosphate modified backbones, such as phosphorothioate backbones.

Exemplary immunostimulatory nucleic acids include but are not limited to the immunostimulatory nucleic acids as listed in Table 1 of US 2003/0087848, which are incorporated herein by reference.

In certain embodiments, an immunostimulatory nucleic acid as taught herein may be a CpG nucleic acid. CpG sequences, while relatively rare in human DNA are commonly found in the DNA of infectious organisms such as bacteria. The human immune system has apparently evolved to recognize CpG sequences as an early warning sign of infection and to initiate an immediate and powerful immune response against invading pathogens without causing adverse reactions frequently seen with other immune stimulatory agents. CpG nucleic acids, relying on this innate immune defense mechanism can utilize a unique and natural pathway for immune therapy.

The phrase "CpG nucleic acid" as used herein refers to a nucleic acid, such as a DNA (e.g. CpG-DNA or CpG DNA), comprising at least one unmethylated CpG dinucleotide.

The term "CpG dinucleotide" refers to a cytosine ("C") followed by guanine ("G") in the linear sequence of bases along its 5' to 3' direction and linked by a phosphate bond ("p"), such as a phosphodiester or a modified phosphorothioate.

The phrase "unmethylated CpG dinucleotide" refers to a CpG dinucleotide wherein at least the cytosine is unmethylated.

Hence, provided herein is a method for *in vitro* differentiation of monocytes to regulatory macrophages, comprising culturing the monocytes in a medium in the presence of a CpG nucleic acid as taught herein, such as a CpG DNA.

A CpG nucleic acid may be double-stranded or single-stranded. In certain embodiments, the CpG nucleic acid may be single-stranded. Advantageously, single-stranded molecules have increased immunostimulatory activity. The entire immunostimulatory nucleic acid, such as CpG nucleic acid, can be unmethylated or portions may be unmethylated but at least the cytosine (C) of the CpG dinucleotide must be unmethylated.

In certain embodiments, an immunostimulatory nucleic acid, such as a CpG nucleic acid, may be represented by at least the formula:
5'-X¹X²CGX³X⁴-3' (SEQ ID NO: 1)
wherein X¹, X², X³, and X⁴ are nucleotides. In an embodiment, X² is adenine, guanine, cytosine, or thymine. In an embodiment, X³ is cytosine, guanine, adenine, or thymine. In other embodiments, X² is adenine, guanine, or thymine and X³ is cytosine, adenine, or thymine.

In certain embodiments, an immunostimulatory nucleic acid, such as a CpG nucleic acid, may be represented by at least the formula:
5'-N¹X¹X²CGX³X⁴N²-3'(SEQ ID NO: 2)
wherein X¹, X², X³, and X⁴ are nucleotides and N is any nucleotide and N¹ and N² are nucleic acid sequences composed of from 0 to 25 nucleotides (Ns) each.

In certain embodiments, an immunostimulatory nucleic acid as taught herein, such as a CpG nucleic acid, may comprise at least 6 nucleotides. In certain embodiments, an immunostimulatory nucleic acid, such as a CpG nucleic acid, may comprise at least 8 nucleotides, such as at least 10, at least 12, at least 15 or at least 18 nucleotides. In certain embodiments, an immunostimulatory nucleic acid, such as a CpG nucleic acid, may comprise at most 100 nucleotides. For instance, an immunostimulatory nucleic acid, such as a CpG nucleic acid, may comprise at most 90 nucleotides, at most 80 nucleotides, at most 70 nucleotides, at most 60 nucleotides, at most 50 nucleotides, at most 40 nucleotides, at most 30 nucleotides, or at most 28 nucleotides.

In certain embodiments, an immunostimulatory nucleic acid as taught herein, such as a CpG nucleic acid, may consist of from 6 to 100 nucleotides. In certain embodiments, an immunostimulatory nucleic acid, such as a CpG nucleic acid, may consist of from 6 to 50, from 6 to 40, from 6 to 30, from 8 to 50, from 8 to 40, from 8 to 30, from 10 to 50, from 10 to 40, from 10 to 30, from 15 to 30, or from 18 to 28 nucleotides. Immunostimulatory nucleic acids consisting of from 6 to 100 nucleotides advantageously facilitate uptake into cells, and hence enhance the efficiency of differentiation of monocytes to regulatory macrophages.

In certain embodiments, an immunostimulatory nucleic acid, such as a CpG nucleic acid, may comprise one or more, such as two or more, e.g. three, four, five, six, seven, eight, nine, ten or more unmethylated CpG dinucleotides.

Exemplary human CpG-DNA is HC4032. Exemplary mouse CpG-DNA is HC4033 (5'-tccatgacgttcctgatgct-3' (SEQ ID NO: 6)). Exemplary control non CpG-DNA is HC4034. Exemplary human and mouse CpG-A oligodeoxynucleotide (ODN) (prototype ODN 2216) is HC4037. Exemplary rabbit CpG-DNA (prototype ODN 2007) is HC4038. Exemplary human and mouse CpG-B oligodeoxynucleotides (prototype ODN 2006) is HC4039. Exemplary rat CpG-B oligodeoxynucleotides is HC4040. Exemplary human and mouse CpG-C oligodeoxynucleotides (prototype ODN 2395) is HC4041. Exemplary non-CpG DNA (rabbit) (prototype ODN 2041) is HC4042. All aforementioned CpG DNA are available from Hycult biotech (The Netherlands).

In certain embodiments, an immunostimulatory nucleic acid, such as a CpG nucleic acid, may contain a CpG motif which is a pathogen-associated molecular pattern (PAMP). In certain embodiments, an immunostimulatory nucleic acid, such as a CpG nucleic acid, may contain a CpG motif mimicking the immunostimulatory effects of bacterial DNA. Optimal CpG motifs differ between animals. In certain embodiments, a CpG nucleic acid as taught herein may contain an optimal human CpG motif or an optimal non-human mammalian CpG motif, e.g. an optimal mouse, rabbit or rat CpG motif. An exemplary human CpG motif is GTCGTT (SEQ ID NO: 3). An exemplary mouse CpG motif is GACGTT (SEQ ID NO: 4).

In certain embodiments, an immunostimulatory nucleic acid, such as a CpG nucleic acid, may comprise a human CpG motif. In certain embodiments, an immunostimulatory nucleic acid, such as a CpG nucleic acid, may comprise a human CpG motif which is GTCGTT (SEQ ID NO: 3).

Nucleic acids having modified backbones, such as phosphorothioate backbones, fall within the class of immunostimulatory nucleic acids. U.S. Pat. No. 5,723,335 and U.S. Pat. No. 5,663,153 issued to Hutcherson, et al. and related PCT publication WO95/26204 describe immune stimulation using phosphorothioate oligonucleotide analogues.

In certain embodiments, an immunostimulatory nucleic acid, such as a CpG nucleic acid, may be a synthetic nucleic acid or a naturally occurring nucleic acid, such as a nucleic acid isolated from bacteria. In certain preferred embodiments, an immunostimulatory nucleic acid, such as a CpG nucleic acid, may be may be a synthetic nucleic acid. Synthetic immunostimulatory nucleic acids, such as synthetic CpG nucleic acid (e.g. a synthetic CpG DNA) advantageously are more efficient to differentiate monocytes into regulatory macrophages in the methods as taught herein.

The term "synthetic nucleic acid" as used herein refers nucleic acids which can be synthesized *de novo* using any of a number of procedures well known in the art.

Synthetic nucleic acids may be obtained *inter alia* by synthesis or recombinant production of such nucleic acids. Chemical synthesis methods include the b-cyanoethyl phosphoramidite method (Beaucage et al., 1981, Tet. Let., 22, 1859); nucleoside H-phosphonate method (Garegg et al., 1986, Tet. Let., 27, 4051-4054; Froehler et al., 1986, Nucl. Acid. Res., 14, 5399-5407; Garegg et al., 1986, Tet. Let., 27, 4055-4058; Gaffney et al., 1988, Tet. Let., 29, 2619-2622). These chemistries can be performed by a variety of automated oligonucleotide synthesizers available in the market. Alternatively, synthetic nucleic acids can be produced by recombinant production on a large scale in plasmids (see Sambrook, T., et al., "Molecular Cloning: A Laboratory Manual", Cold Spring Harbor laboratory Press, New York, 1989), and separated into smaller pieces (i.e. isolated) or administered whole.

The immunostimulatory nucleic acids can be prepared from existing nucleic acid sequences (e.g., genomic or cDNA) using known techniques, such as those employing restriction enzymes, exonucleases or endonucleases.

In certain embodiments of the methods as taught herein, the concentration of the immunostimulatory nucleic acid, such as CpG nucleic acid, in the medium may be from 0.01 to 200 µg per ml of the medium. For example, the concentration of the immunostimulatory nucleic acid, such as CpG nucleic acid, in the medium may be from 0.025 to 175 µg/ml, from 0.050 to 150 µg/ml, such as from 0.075 to 125 µg/ml, from 0.10 to 100 µg/ml, from 0.25 to 75 µg/ml, from 0.50 to 50 µg/ml, or from 0.75 to 25 µg/ml of the medium. Preferably, the concentration of the immunostimulatory nucleic acid, such as CpG nucleic acid, in the medium is from 0.1 to 20 µg per ml of the medium. For instance, the concentration of the immunostimulatory nucleic acid, such as CpG nucleic acid, in the medium may be from 0.1 to 10 µg, from 1 to 20 µg, from 1 to 10 µg, or from 5 to 10 µg per ml of the medium.

For example, the concentration of the immunostimulatory nucleic acid, such as CpG nucleic acid, in the medium is 0.1 µg/ml, 0.2 µg/ml, 0.3 µg/ml, 0.4 µg/ml, 0.5 µg/ml, 0.6 µg/ml, 0.7 µg/ml, 0.8 µg/ml, 0.9 µg/ml, or 1 µg/ml, 2 µg/ml, 3 µg/ml, 4 µg/ml, 5 µg/ml, 6 µg/ml, 7 µg/ml, 8 µg/ml, 9 µg/ml, 10 µg/ml, 11 µg/ml, 12 µg/ml, 13 µg/ml, 14 µg/ml, 15 µg/ml, 16 µg/ml, 17 µg/ml, 18 µg/ml, 19 µg/ml, or 20 µg/ml of the medium.

In certain embodiments of the methods as taught herein, the concentration of the monocytes in the medium, and the concentration of the immunostimulatory nucleic acid, such as CpG nucleic acid, in the medium, may be as shown in Table 1 (each row of Table 1 providing an example of such an embodiment).

**Table 1: Exemplary embodiments of the medium used in the present methods**

| **Concentration of monocytes in the medium (cells per ml of the medium)** | **Concentration of the immunostimulatory nucleic acid, such as CpG nucleic acid, in the medium (µg per ml of the medium)** |
|---|---|
| from about 1 x 10⁴ to about 1 x 10⁶ cells/ml, e.g. | from 0.1 to 20 µg/ml |
| from about 1 x 10⁵ to about 2 x 10⁵ cells/ml | |
| from about 1 x 10⁴ to about 1 x 10⁶ cells/ml, e.g. from about 1 x 10⁵ to about 2 x 10⁵ cells/ml | from 0.1 to 10 µg/ml |
| from about 1 x 10⁴ to about 1 x 10⁶ cells/ml, e.g. from about 1 x 10⁵ to about 2 x 10⁵ cells/ml | from 1 to 20 µg/ml |
| from about 1 x 10⁴ to about 1 x 10⁶ cells/ml, e.g. from about 1 x 10⁵ to about 2 x 10⁵ cells/ml | from 1 to 10 µg/ml |
| from about 1 x 10⁴ to about 1 x 10⁶ cells/ml, e.g. from about 1 x 10⁵ to about 2 x 10⁵ cells/ml | from 5 to 10 µg/ml |
| from about 5 x 10⁴ to about 2 x 10⁵ cells/ml, e.g. from about 5 x 10⁴ to about 1 x 10⁵ cells/ml | from 0.1 to 20 µg/ml |
| from about 5 x 10⁴ to about 2 x 10⁵ cells/ml, e.g. from about 5 x 10⁴ to about 1 x 10⁵ cells/ml | from 0.1 to 10 µg/ml |
| from about 5 x 10⁴ to about 2 x 10⁵ cells/ml, e.g. from about 5 x 10⁴ to about 1 x 10⁵ cells/ml | from 1 to 20 µg/ml |
| from about 5 x 10⁴ to about 2 x 10⁵ cells/ml, e.g. from about 5 x 10⁴ to about 1 x 10⁵ cells/ml | from 1 to 10 µg/ml |
| from about 5 x 10⁴ to about 2 x 10⁵ cells/ml, e.g. from about 5 x 10⁴ to about 1 x 10⁵ cells/ml | from 5 to 10 µg/ml |

### Isolating step

In certain embodiments of the methods as taught herein, the method may comprise the prior step of isolating the monocytes. In certain embodiments, the method may comprise the prior step of isolating the monocytes from a biological sample, such as whole blood, bone marrow, spleen and/or lung. In certain embodiments, the method may comprise the prior step of isolating the monocytes from whole blood and/or bone marrow.

The isolation step may be performed using conventional methods, such as any suitable technique as described herein for the isolation of monocytes from whole blood, bone marrow, spleen and/or lung.

In certain embodiments, the method may comprise the prior step of isolating the monocytes from a biological sample of a subject, thereby obtaining monocytes or a cell population comprising monocytes.

In certain embodiments, a cell population comprising monocytes may comprise at least 60% of monocytes. For instance, the cell population comprising monocytes may comprise at least 65% of monocytes, e.g. at least 70%, at least 75%, at least 80%, or at least 85% of monocytes. Preferably, the cell population comprising monocytes may comprise at least 90% of monocytes. More preferably, the cell population comprising monocytes may comprise at least 95% of monocytes, such as 95%, 96%, 97%, 98%, 99% or 100% of monocytes.

### Culturing step

As mentioned above, in certain embodiments, the methods as taught herein comprise culturing the monocytes in a medium in the presence of an immunostimulatory nucleic acid, such as a CpG nucleic acid.

The phrase "culturing the monocytes in a medium in the presence of an immunostimulatory nucleic acid" refers to contacting the monocytes with or exposing the monocytes to a medium comprising an immunostimulatory nucleic acid. The monocytes are contacted with the medium comprising an immunostimulatory nucleic acid to ensure that the immunostimulatory nucleic acid can trigger differentiation of the monocytes to regulatory macrophages.

The term "contacting" as used herein refers to bringing together, either directly or indirectly, one or more molecules, components or materials with another, thereby facilitating interactions there between. Typically, monocytes may be contacted with an immunostimulatory nucleic acid, or in other words, monocytes may be exposed to an immunostimulatory nucleic acid, by means of its inclusion in the medium in which the monocytes are cultured.

In an embodiment, the culturing step is performed in the presence of a medium, commonly a liquid cell culture medium. Typically, the medium will comprise a basal medium formulation as known in the art. Many basal media formulations (available, e.g., from the American Type Culture Collection, ATCC; or from Invitrogen, Carlsbad, California) can be used to culture the cells herein. Well-known liquid cell culture media include Roswell Park Memorial Institute (RPMI) medium, Eagle's Minimum Essential Medium (MEM), Dulbecco's Modified Eagle's Medium (DMEM), alpha modified Minimum Essential Medium (alpha-MEM), Basal Medium Essential (BME), BGJb, F-12 Nutrient Mixture (Ham), Iscove's Modified Dulbecco's Medium (IMDM), or X-Vivo-10 serum free medium (clinical grade), available from Invitrogen or Cambrex (New Jersey), and modifications and/or combinations thereof. Compositions of the above media are generally known in the art and it is within the skill of one in the art to modify or modulate concentrations of media and/or media supplements as necessary for the cells cultured.

Such basal media formulations contain ingredients necessary for mammal cell development, which are known *per se.* By means of illustration and not limitation, these ingredients may include inorganic salts (in particular salts containing Na, K, Mg, Ca, Cl, P and possibly Cu, Fe, Se and Zn), physiological buffers (e.g., HEPES, bicarbonate), nucleotides, nucleosides and/or nucleic acid bases, ribose, deoxyribose, amino acids, vitamins, antioxidants (e.g., glutathione) and sources of carbon (e.g. glucose, sodium pyruvate, sodium acetate), etc.

For use in culture, the (cell culture) medium can be supplied with one or more further components. For example, additional supplements can be used to supply the cells with the necessary trace elements and substances for optimal growth and expansion. Such supplements include insulin, transferrin, selenium salts, and combinations thereof. These components can be included in a salt solution such as, but not limited to, Hanks' Balanced Salt Solution (HBSS), Earle's Salt Solution. Further antioxidant supplements may be added, e.g., β-mercaptoethanol. While many basal media already contain amino acids, some amino acids may be supplemented later, e.g., non-essential amino acids (NEAA) and/or L-glutamine, which is known to be less stable when in solution. A medium may be further supplied with antibiotic and/or antimycotic compounds, such as, typically, mixtures of penicillin and streptomycin.

In certain embodiments of the methods as taught herein, the (cell culture) medium, such as RPMI medium or DMEM, comprises an immunostimulatory nucleic acid as taught herein. In certain embodiments of the methods as taught herein, the (cell culture) medium, such as RPMI medium or DMEM, may further comprise serum, such as Fetal bovine serum (FBS) or Fetal calf serum (FCS). In certain embodiments, the serum may be heat inactivated as known in the art, particularly to remove the complement.

The serum as intended herein may be human serum, i.e., obtained from a single human subject or from a plurality of human subjects (e.g., serum mixed pool).

The serum may be preferably unprocessed serum, i.e., serum derived by separation from whole blood and not subjected to downstream processing steps which alter its chemical, biochemical, or cellular composition, other than optional heat inactivation, storage (cryogenic or non-cryogenic), sterilisation, freeze-drying and/or filtration.

The term "serum" may in certain embodiments specifically exclude processed serum, i.e., serum subjected after its separation from whole blood to one or more processing steps which alter its composition, specifically its chemical, biochemical, or cellular composition.

The serum may be used directly in the methods as taught herein. The serum can also be appropriately stored for later use (e.g., for shorter time periods, e.g., up to about 1-2 weeks, at a temperature above the freezing points of serum, but below ambient temperature, this temperature will usually be about 4°C to 5°C; or for longer times by freeze storage, usually at between about - 16°C and about -24°C, or at between about -70°C and about -80°C).

Optionally, the serum may also be sterilized prior to storage or use, using conventional microbiological filters, preferably with pore size of 0.2 µm or smaller.

In certain embodiments, the methods may employ serum which is autologous to the subject to be treated, and hence to the monocytes to be differentiated in the medium comprising the serum. The term "autologous" with reference to serum denotes that the serum is obtained from the same subject to be contacted or treated with the regulatory macrophages obtained by the methods as taught herein. In certain embodiments, the methods may employ serum which is "homologous" or "allogeneic" to the subject to be treated, i.e., obtained from one or more (pooled) subjects other than the subject to be contacted or treated with the regulatory macrophages obtained by the methods as taught herein. In certain embodiments, the methods may employ a mixture of autologous and homologous (allogeneic) sera as defined above. Particularly intended herein is human serum, more preferably autologous human serum for the *in vitro* differentiation of monocytes to regulatory macrophages.

The serum can be heat inactivated as known in the art, particularly to remove the complement. Where the present methods employ serum autologous to the cells cultured in the presence thereof, it may be unnecessary to heat inactivate the serum. Where the serum is at least partly allogeneic to the cultured cells, it may be advantageous to heat inactivate the serum.

In certain embodiments, the method may further comprise culturing the monocytes or a cell population comprising monocytes in a medium in the presence of serum. In certain embodiments, the method may comprise culturing the monocytes or a cell population comprising monocytes in a medium in the presence of an immunostimulatory nucleic acid and serum.

In certain embodiments of the methods as taught herein, the concentration of the serum in the medium may be at most 10% (v/v), e.g. from 0.1% to 10% (v/v), for example the concentration of the serum in the medium may be 0.5% (v/v), 1.0% (v/v), 5.0% (v/v) or 10% (v/v).

In certain embodiments of the methods as taught herein, the (cell culture) medium, such as RPMI medium or DMEM, may further comprise a growth factor, such as macrophage colony-stimulating factor (M-CSF). In certain embodiments, the (cell culture) medium, such as RPMI medium or DMEM, may further comprise M-CSF. M-CSF advantageously increases the viability of the monocytes and/or cells derived thereof including the regulatory macrophages as taught herein.

In certain embodiments, the method may further comprise culturing the monocytes or a cell population comprising monocytes in a medium in the presence of a growth factor, such as M-CSF. Hence, in certain embodiments, the method may further comprise culturing the monocytes or a cell population comprising monocytes in a medium in the presence of M-CSF. In certain embodiments, the method may thus comprise culturing the monocytes or a cell population comprising monocytes in a medium in the presence of an immunostimulatory nucleic acid and a growth factor, such as M-CSF. In certain embodiments, the method may comprise culturing the monocytes or a cell population comprising monocytes in a medium in the presence of an immunostimulatory nucleic acid; serum; and a growth factor, such as M-CSF.

In certain embodiments of the methods as taught herein, the concentration of M-CSF in the medium may be from 0.1 to 500 ng per ml of the medium. In certain embodiments, the concentration of M-CSF in the medium may be from 0.1 to 100 ng per ml of the medium. For example, the concentration of M-CSF in the medium may be from 1 to 10 ng, from 1 to 20 ng, from 1 to 50 ng, from 5 to 100 ng, or from 10 to 100 ng per ml of the medium.

A skilled person appreciates that serum is a complex biological composition, which may comprise one or more growth factors, cytokines or hormones. Hence, it is intended that M-CSF is provided in addition to, i.e., exogenously to or in supplement to, the serum.

In certain embodiments of the methods as taught herein, the monocytes or cell population comprising monocytes may be cultured in a medium in the presence of an immunostimulatory nucleic acid (i.e. monocytes and immunostimulatory nucleic acid may be contacted) for at least 1 hour, such as for at least 6 hours, for at least 12 hours, or for at least 24 hours. For instance, the monocytes or cell population comprising monocytes may be cultured in a medium in the presence of an immunostimulatory nucleic acid (i.e. monocytes and immunostimulatory nucleic acid may be contacted) for at least 48 hours (2 days), for at least 56 hours, for at least 60 hours, for at least 72 hours (3 days), for at least 96 hours (4 days), for at least 120 hours (5 days), for at least 144 hours (6 days), for at least 168 hours (7 days), or for at least 192 hours (8 days).

In certain embodiments, the monocytes or cell population comprising monocytes may be cultured in a medium in the presence of an immunostimulatory nucleic acid (i.e. monocytes and immunostimulatory nucleic acid may be contacted) for at most 240 hours (10 days), such as for at most 216 hours (9 days), for at most 192 hours (8 days), for at most 168 hours (7 days), or for at most 144 hours (6 days). For instance, the monocytes or cell population comprising monocytes may be cultured in a medium in the presence of an immunostimulatory nucleic acid (i.e. monocytes and immunostimulatory nucleic acid may be contacted) for at most 120 hours (5 days), for at most 96 hours (4 days), for at most 72 hours (3 days), or for at most 48 hours (2 days).

In certain embodiments, the monocytes or cell population comprising monocytes may be cultured in a medium in the presence of an immunostimulatory nucleic acid (i.e. monocytes and immunostimulatory nucleic acid may be contacted) for 24 hours (1 day) to 240 hours (10 days). For instance, the monocytes or cell population comprising monocytes may be cultured in a medium in the presence of an immunostimulatory nucleic acid (i.e. monocytes and immunostimulatory nucleic acid may be contacted) for 24 hours (1 day) to 192 hours (8 days), for 24 hours (1 day) to 144 hours (6 days), for 24 hours (1 day) to 96 hours (4 days), for 24 hours (1 day) to 72 hours (3 days), or for 48 hours (2 days) to 144 hours (6 days), for 48 hours (2 days) to 120 hours (5 days), for 48 hours (2 days) to 96 hours (4 days), or for 48 hours (2 days) to 72 hours (3 days).

In certain embodiments, the monocytes or cell population comprising monocytes may be cultured in a medium in the presence of an immunostimulatory nucleic acid for 24 hours (1 days), for 36 hours, for 48 hours (2 days), for 56 hours, for 60 hours, for 72 hours (3 days), for 96 hours (4 days), for 120 hours (5 days), for 144 hours (6 days), for 168 hours (7 days), for 192 hours (8 days), for 216 hours (9 days), or for 240 hours (10 days).

Time, as used herein, is expressed in hours or days. It is to be noted however that time may also be expressed in other units such as minutes (min).

The terms "time", "time period", and "time duration" may be used interchangeably.

In certain embodiments, the monocytes or cell population comprising monocytes may be cultured in a medium in the presence of an immunostimulatory nucleic acid (i.e. monocytes and immunostimulatory nucleic acid may be contacted) at a temperature ranging from about 30°C to about 40°C. In certain embodiments of the methods as taught herein, the monocytes or cell population comprising monocytes may be cultured in a medium in the presence of an immunostimulatory nucleic acid at a temperature of about 37°C.

Temperature, as used herein, is expressed in degrees Celsius (°C). It is to be noted however that temperature may also be expressed in any other suitable unit such as Kelvin (K).

In certain embodiments, the methods as taught herein may be performed at a pH ranging from about 6 to about 8. In certain embodiments, the methods as described herein may be performed at a near neutral pH (i.e., pH of about 7).

### Method details

In an embodiment, the monocytes or cell population comprising monocytes may be continuously contacted with an immunostimulatory nucleic acid, i.e. the immunostimulatory nucleic acid would be included in all media in which the monocytes or cell population comprising monocytes and/or cells derived thereof (e.g. regulatory macrophages or cell population comprising regulatory macrophages) are cultured during the method. Typically, the immunostimulatory nucleic acid may be supplied at substantially identical concentration in all (fresh) media used for culturing the monocytes or cell population comprising monocytes and/or cells derived thereof during the method.

Hence, in certain embodiments, the methods as taught herein may comprise:
(a) isolating monocytes from a biological sample (e.g. whole blood) of a subject, thereby obtaining monocytes or a cell population comprising monocytes;
(b) adding the monocytes or the cell population comprising monocytes to a medium comprising an immunostimulatory nucleic acid, thereby obtaining a monocyte-medium mixture; and
(c) culturing the monocyte-medium mixture, thereby obtaining regulatory macrophages or a cell population comprising regulatory macrophages.

In another embodiment, the method may comprise the step of (a') adding the monocytes or cell population comprising monocytes to a medium in the absence of (i.e. without) an immunostimulatory nucleic acid; and optionally (a") culturing the monocytes or cell population comprising monocytes in a medium in the absence of an immunostimulatory nucleic acid. After step (a') or the optional step (a"), an immunostimulatory nucleic acid may be added to the monocyte-medium mixture to continue with the method step (c). Alternatively, the medium without an immunostimulatory nucleic acid (optionally containing non-adherent cells) may be replaced by a medium comprising an immunostimulatory nucleic acid to continue with the method step (c).

Accordingly, in certain embodiments, the methods as taught herein may comprise:
(a) isolating monocytes from a biological sample (e.g. whole blood) of a subject, thereby obtaining monocytes or a cell population comprising monocytes;
(a') adding the monocytes or cell population comprising monocytes to a medium without an immunostimulatory nucleic acid, thereby obtaining a monocyte-medium mixture;
(a") optionally culturing the monocyte-medium mixture;
(b') adding an immunostimulatory nucleic acid to the monocyte-medium mixture or (b") replacing the medium without an immunostimulatory nucleic acid (optionally containing non-adherent cells) by a medium comprising an immunostimulatory nucleic acid; and
(c) culturing the monocyte-medium mixture, thereby obtaining regulatory macrophages or a cell population comprising regulatory macrophages.

In certain embodiments of the methods as taught herein, the monocytes or cell population comprising monocytes may be cultured in a medium without (in the absence of) an immunostimulatory nucleic acid (step (a")) for at most 6 hours. For instance, the monocytes or cell population comprising monocytes may be cultured in a medium without an immunostimulatory nucleic acid for at most 5 hours, for at most 4 hours, for at most 3 hours, for at most 2 hours, or for at most 1 hour.

In certain embodiments of the methods as taught herein, the monocytes or cell population comprising monocytes may be cultured in a medium without (in the absence of) an immunostimulatory nucleic acid (step (a")) for 1 hour to 6 hours. For instance, the monocytes or cell population comprising monocytes may be cultured in a medium in the absence of an immunostimulatory nucleic acid for 3 hours to 6 hours.

In certain embodiments, macrophage colony-stimulating factor (M-CSF) may be added to the medium without an immunostimulatory nucleic acid. M-CSF advantageously increases the viability of the monocytes.

In certain embodiments of the methods as taught herein, the monocytes or cell population comprising monocytes may be cultured in a medium without an immunostimulatory nucleic acid (e.g. step (a")) but comprising M-CSF for at most 2 days. For instance, the monocytes or cell population comprising monocytes may be cultured in a medium without an immunostimulatory nucleic acid but comprising M-CSF for at most 1 day, for at most 12 hours, for at most 6 hours, for at most 3 hours, or for at most 1 hour.

In certain embodiments of the methods as taught herein, the monocytes or cell population comprising monocytes may be cultured in a medium without (in the absence of) an immunostimulatory nucleic acid (e.g. step (a")) for 1 hour to 2 days. For instance, the monocytes or cell population comprising monocytes may be cultured in a medium in the absence of an immunostimulatory nucleic acid for 1 day to 2 days. Culturing the monocytes or cell population comprising monocytes in the presence of M-CSF advantageously allows increasing the viability of the monocytes as compared to culturing in the absence of M-CSF.

In certain embodiments, the monocytes or cell population comprising monocytes may be plated for culturing (e.g. in step in step (a") or step (c)) at between about 1 x 10⁴ and about 1 x 10⁶ cells per ml of the medium, preferably at between about 5 x 10⁴ and about 2 x 10⁵ cells per ml of the medium, more at about 1 x 10⁵ cells per ml of the medium.

In an embodiment, the culture vessel may provide for a plastic surface to enable cell adherence. In another embodiment, the surface may be a glass surface. In yet another embodiment, the surface may be coated with an appropriate material conducive to adherence and growth of cells, e.g., Matrigel(R), laminin or collagen.

Typically, the monocytes or cell population comprising monocytes may be cultured in a medium in the presence of an immunostimulatory nucleic acid (e.g. in culturing step (c)) for a period as described herein, preferably for 24 hours (1 day) to 240 hours (10 days).

In an embodiment, following the culturing step (e.g. step (c)), the method may comprise collecting the regulatory macrophages or cell population comprising regulatory macrophages.

In certain embodiments, the cell population comprising regulatory macrophages may comprise at least 60% of regulatory macrophages. For instance, the cell population comprising regulatory macrophages may comprise at least 65% of regulatory macrophages, e.g. at least 70%, at least 75%, at least 80%, or at least 85% of regulatory macrophages. Preferably, the cell population comprising regulatory macrophages may comprise at least 90% of regulatory macrophages. More preferably, the cell population comprising regulatory macrophages may comprise at least 95% of regulatory macrophages, such as 95%, 96%, 97%, 98%, 99% or 100% of regulatory macrophages.

In certain embodiments, the method may further comprise culturing the regulatory macrophages or cell population comprising regulatory macrophages in the presence of one or more antigens prior to collection of the cells. Since regulatory macrophages express high levels of MHC-II molecules, regulatory macrophages or a cell population comprising regulatory macrophages may be cultured prior to their collection in the presence of one or more antigens. Advantageously, this may allow obtaining regulatory macrophages which present antigens and are capable of inducing regulatory T-cell responses. In certain embodiments, the antigen may be an allergen.

Suitable non-limiting examples of an allergen include one or more of dust mites (e.g., house dust mite (HDM), Der f 1, or Der p 1), cat (e.g., Fel d 1), dog (e.g., Can f 1), and cockroach (e.g., Bla g1 or Bla g 2).

In certain embodiments, the regulatory macrophages or cell population comprising regulatory macrophages may be cultured in the presence of one or more antigens for at least 1 hour prior to collection of the cells. For instance, the regulatory macrophages or cell population comprising regulatory macrophages may be cultured in the presence of one or more antigens for at least 3 hours prior to collection of the cells, e.g. for at least 6 hours, at least 12 hours, or at least 24 hours prior to collection of the cells. In an embodiment, one or more antigens may be added during the culturing step (e.g. step (c)) to the medium, for instance at least 1 hour prior to collection of the cells, e.g. for at least 3 hours, at least 6 hours, at least 12 hours, at least 18 hours, or at least 24 hours prior to collection of the cells.

In certain embodiments, the regulatory macrophages or cell population comprising regulatory macrophages may be cultured in the presence of one or more antigens for 1 hour to 24 hours prior to collection of the cells. For instance, the regulatory macrophages or cell population comprising regulatory macrophages may be cultured in the presence of one or more antigens for 3 hours to 18 hours prior to collection of the cells, e.g. for 6 hours to 12 hours prior to collection of the cells. In an embodiment, one or more antigens may be added during the culturing step (e.g. step (c)) to the medium, for instance 1 hour to 24 hours prior to collection of the cells, e.g. for 3 hours to 18 hours or for 6 hours to 12 hours prior to collection of the cells.

In certain embodiments, the regulatory macrophages or cell population comprising regulatory macrophages may be collected as an adherent monolayer on a surface and/or may be in cell suspension (i.e., non-adherent cells). In an embodiment, adherent and non-adherent cells may be collected. In an embodiment, only adherent or non-adherent cells may be collected.

In an embodiment, adherent cells may be collected using mechanical dissociation, e.g. flushing the cells with a suitable aqueous solution, such as a salt solution. This allows collecting the adherent cells in an easy and non-aggressive manner. In another embodiment, adherent cells may be collected by detachment of cells using a bivalent ion chelator (e.g., EDTA or EGTA) and/or trypsin or suitable protease; and re-suspending the detached cells in a suitable aqueous solution.

In certain embodiments, the aqueous solution may be a salt solution (e.g., phosphate buffered saline (PBS) or Hanks' Balanced Salt solution (HBSS)), a salt solution (e.g., PBS or HBSS) containing a bivalent ion chelator (e.g., EDTA or EGTA), or cell culture medium. Preferably, the aqueous solution is a salt solution such as PBS.

Typically, after collection, the regulatory macrophages or cell population comprising regulatory macrophages may be administrated immediately to the patient. For instance, the regulatory macrophages or cell population comprising regulatory macrophages may be administrated to the patient at most 1 min after collection of the cells, e.g., the regulatory macrophages or cell population comprising regulatory macrophages may be administrated to the patient at most 5 min, at most 10 min, at most 15 min, at most 30 min, or at most 60 min after collection of the cells.

Alternatively, the regulatory macrophages or cell population comprising regulatory macrophages may be transported and/or stored for later use. For instance, the regulatory macrophages or cell population comprising regulatory macrophages may be transported and/or stored for short time periods, such as for 6 hours, for 12 hours, for 18 hours, for 24 hours or more, such as for 2 days, for 3 days, for 4 days, for 5 days, for 6 days or more, e.g., up to about 1 to 2 weeks, for instance at a temperature below ambient temperature. This temperature will usually be at about 4°C to 7°C.

The methods as taught herein yield regulatory macrophages, or cell populations comprising such, with superior characteristics, such as in particular high expression of IL-10, which regulatory macrophages, or cell populations comprising such, are suited for prophylactic or therapeutic treatment of an inflammatory respiratory disease, in particular a chronic respiratory disease, such as asthma or COPD.

### Regulatory macrophages

The term "regulatory macrophages" as used herein refers to a cell population comprising or consisting of *in vitro* differentiated regulatory macrophages.

A further aspect relates to regulatory macrophages obtainable or obtained by a method as defined herein. Hence, certain embodiments provide regulatory macrophages obtainable or obtained by a method for *in vitro* differentiation of monocytes to regulatory macrophages, comprising culturing the monocytes in a medium in the presence of an immunostimulatory nucleic acid, such as a CpG nucleic acid. The (*in vitro* differentiated) regulatory macrophages as taught herein advantageously are characterized by the ability to produce IL-10.

In certain embodiments of the methods or regulatory macrophages as taught herein, the regulatory macrophages as taught herein may be characterized by production of Interleukin-10 (IL-10). A further aspect relates to (*in vitro* differentiated) regulatory macrophages characterized by production of Interleukin-10.

In certain embodiments of the methods or regulatory macrophages as taught herein, the regulatory macrophages as taught herein may be characterized by almost no or no production of Interleukin-12 (IL-12). In certain embodiments, the regulatory macrophages as taught herein may be characterized by production of IL-10 and by almost no or no production of IL-12. In certain embodiments, the regulatory macrophages as taught herein may be characterized by a ratio of IL-10 (e.g., IL-10 concertation in the medium) to IL-12 (e.g., IL-12 concentration in the medium) of at least 2, such as at least 5, at least 10, at least 50, at least 100, at least 500, at least 1000 or more, preferably at least 5000, at least 10000, at least 50000, at least 100000, or more.

In certain embodiments of the methods or regulatory macrophages as taught herein, the regulatory macrophages as taught herein may have similar properties as *in vivo* regulatory macrophages. For instance, *in vivo* regulatory macrophages have been described in Mosser and Edwards (2008, Nat. Rev. Immunol., 8, 958-969). In certain embodiments, the regulatory macrophages as taught herein may have anti-inflammatory properties. In certain embodiments, the regulatory macrophages as taught herein may have hypersuppressive properties.

In certain embodiments of the methods or regulatory macrophages as taught herein, the regulatory macrophages as taught herein may be characterized by production of Transforming Growth Factor beta (TGF-β).

In certain embodiments of the methods or regulatory macrophages as taught herein, the regulatory macrophages as taught herein may be characterized by expression of co-stimulatory molecules (e.g., CD80 and/or CD86).

In certain embodiments, the regulatory macrophages as taught herein may be characterized by expression of CD64 (CD64⁺).

In preferred embodiments of the methods or regulatory macrophages as taught herein, the regulatory macrophages as taught herein may be living (viable) cells. Preferably, the regulatory macrophages as taught herein are functional cells.

In certain embodiments of the methods or regulatory macrophages as taught herein, the regulatory macrophages as taught herein may be autologous to the subject to be treated. The term "autologous" with reference to regulatory macrophages denotes that the regulatory macrophages are obtained from monocytes isolated from the same subject to be contacted or treated with the regulatory macrophages. In certain embodiments, the regulatory macrophages may be "homologous" or "allogeneic" to the subject to be treated, i.e., obtained from monocytes isolated form one or more (pooled) subjects other than the subject to be contacted or treated with the regulatory macrophages. The regulatory macrophages may also comprise a mixture of autologous and homologous (allogeneic) regulatory macrophages as defined above. Preferably, the regulatory macrophages as taught herein are autologous to the subject to be treated.

A further aspect relates to the use of an immunostimulatory nucleic acid as taught herein, such as a CpG nucleic acid, for preparing the regulatory macrophages as taught herein.

A further aspect relates to (*in vitro* differentiated) regulatory macrophages as taught herein for use in the treatment (including throughout the present specification therapeutic and/or preventative measures) of an inflammatory respiratory disease, in particular a chronic respiratory disease, such as asthma or COPD. Hence, certain embodiments provide regulatory macrophages for use in treating an inflammatory respiratory disease, in particular a chronic respiratory disease, such as asthma or COPD, wherein the regulatory macrophages are obtainable or obtained by a method as taught herein. Certain embodiments thus provide regulatory macrophages for use in treating an inflammatory respiratory disease, in particular a chronic respiratory disease, such as asthma or COPD, wherein the regulatory macrophages are obtainable or obtained by a method for *in vitro* differentiation of monocytes to regulatory macrophages, the method comprising culturing the monocytes in a medium in the presence of an immunostimulatory nucleic acid, such as a CpG nucleic acid. The *in vitro* differentiated regulatory macrophages as taught herein advantageously produce increased levels of IL-10 and have excellent hypersuppressive properties, thereby making them particularly useful in the treatment of inflammatory respiratory diseases, in particular chronic respiratory diseases, such as asthma or COPD.

A related aspect provides a method of treating an inflammatory respiratory disease, in particular a chronic respiratory disease, such as asthma or COPD, in a subject in need of such treatment comprising administering to said subject a therapeutically or prophylactically effective amount of (*in vitro* differentiated) regulatory macrophages as taught herein. Accordingly, certain embodiments provide a method of treating an inflammatory respiratory disease, in particular a chronic respiratory disease, such as asthma or COPD, in a subject in need of such treatment comprising administering to said subject a therapeutically or prophylactically effective amount of regulatory macrophages obtainable or obtained by a method as taught herein. Certain embodiments thus provide a method of treating an inflammatory respiratory disease, in particular a chronic respiratory disease, such as asthma or COPD, in a subject in need of such treatment comprising administering to said subject a therapeutically or prophylactically effective amount of regulatory macrophages obtainable or obtained by a method for *in vitro* differentiation of monocytes to regulatory macrophages, the method comprising culturing the monocytes in a medium in the presence of an immunostimulatory nucleic acid, such as a CpG nucleic acid.

A related aspect provides the use of (*in vitro* differentiated) regulatory macrophages as taught herein for the manufacture of a medicament for the treatment of an inflammatory respiratory disease, in particular a chronic respiratory disease, such as asthma or COPD. Thus, certain embodiments provide the use of regulatory macrophages for the manufacture of a medicament for the treatment of an inflammatory respiratory disease, in particular a chronic respiratory disease, such as asthma or COPD, wherein the regulatory macrophages are obtainable or obtained by a method as taught herein. Certain embodiments thus provide the use of regulatory macrophages for the manufacture of a medicament for the treatment of an inflammatory respiratory disease, in particular a chronic respiratory disease, such as asthma or COPD, wherein the regulatory macrophages are obtainable or obtained by a method for *in vitro* differentiation of monocytes to regulatory macrophages, the method comprising culturing the monocytes in a medium in the presence of an immunostimulatory nucleic acid, such as a CpG nucleic acid.

The terms "respiratory disease", "respiratory tract disease" or "airway disease" may be used interchangeably herein and generally refers to any pathological condition affecting the organs of the respiratory tract.

The term "respiratory tract" generally refers to the part of the anatomy involved with the process of respiration. The respiratory tract is typically divided into: (1) the upper respiratory tract comprising the nose and nasal passages, paranasal sinuses, throat or pharynx, and the voice box or larynx; and (2) the lower respiratory tract comprising trachea, bronchi, bronchioles, and the lungs comprising respiratory bronchioles, alveolar ducts, alveolar sacs, and alveoli.

In certain embodiments, the respiratory disease may be a disease of the lower respiratory tract (i.e. lower respiratory tract disease). In certain embodiments, the inflammatory respiratory disease may be any inflammatory disease of the lower respiratory tract (i.e. inflammatory lower respiratory tract disease).

The term "inflammatory respiratory disease" as used herein refers to any respiratory disease involving inflammation. The term "inflammation" as used herein has its meaning as known in the art and generally refers to a biological response of the body involving immune cells.

In certain embodiments, the inflammatory respiratory disease may be selected from the group consisting of asthma, chronic obstructive pulmonary disease (COPD), chronic bronchitis, acute respiratory distress syndrome (ARDS), pneumonia, respiratory distress of prematurity, cystic fibrosis, pulmonary fibrosis, and pulmonary sarcoidosis.

The term "asthma" refers to an inflammatory disease of the airways of the lungs. Usually, asthma is characterized by respiratory symptoms such as wheeze, shortness of breath, chest lightness and cough that vary over time and in intensity, together with variable expiratory airflow limitation.

Asthma is clinically classified according to the frequency of symptoms, forced expiratory volume in one second (FEV₁), and peak expiratory flow rate. Table 2 provides an overview of the clinical classification of asthma for subjects of at least 12 years old.

**Table 2: Clinical classification of asthma for subjects of at least 12 years old**

| **Severity** | **Symptom frequency** | **Night time symptoms** | **% FEV₁ of predicted** | **FEV₁ Variability** | **SABA use** |
|---|---|---|---|---|---|
| Intermittent | ≤2/week | ≤2/month | >80% | <20% | ≤2 days/week |
| Mild persistent | >2/week | 3-4/month | ≥80% | 20-30% | >2 days/week |
| Moderate persistent | Daily | >1/week | 60-80% | >30% | daily |
| Severe persistent | Continuously | Frequent (7 times/week) | <60% | >30% | ≥twice/day |

| | | | | | |
|---|---|---|---|---|---|
| FEV₁: forced expiratory volume in one second; SABA: short-acting beta2-adrenoceptor agonists | | | | | |

The term "chronic obstructive pulmonary disease (COPD)" refers to an obstructive lung disease characterized by persistent respiratory symptoms and airflow limitation that is due to airway and/or alveolar abnormalities. COPD is usually caused by significant exposure to noxious particles or gases. Tobacco smoking is the most common cause of COPD.

In certain embodiments, the COPD may be chronic bronchitis.

The term "chronic bronchitis" refers to a chronic disease of the lung that is defined based on the following functional criterion: FEV₁/forced vital capacity (FVC) < 70% after bronchodilation.

The term "acute respiratory distress syndrome (ARDS)" refers to a medical condition occurring in critically ill patients characterized by widespread inflammation in the lungs. ARDS may be characterized by diffuse injury to cells which form the alveolar barrier, surfactant dysfunction, activation of the innate immune response, and abnormal coagulation.

The term "pneumonia" refers to an inflammatory condition of the lung affecting primarily the microscopic air sacs (alveoli). Typical signs and symptoms include a varying severity and combination of productive or dry cough, chest pain, fever, and trouble breathing, depending on the underlying cause.

The terms "respiratory distress of prematurity", "infant respiratory distress syndrome (IRDS)", "neonatal respiratory distress syndrome", "respiratory distress syndrome of newborn", or "surfactant deficiency disorder (SDD)" refer to a syndrome in premature infants caused by developmental insufficiency of pulmonary surfactant production and structural immaturity in the lungs.

The term "cystic fibrosis" refers to a genetic disorder that affects mostly the lungs, but also the pancreas, liver, kidneys, and intestine, and that is caused by the presence of mutations in both copies of the gene for the cystic fibrosis transmembrane conductance regulator (CFTR) protein. Long-term issues include difficulty breathing and coughing up mucus as a result of frequent lung infections.

The term "pulmonary fibrosis" refers to a respiratory disease in which scars are formed in the lung tissues, leading to serious breathing problems. Scar formation, the accumulation of excess fibrous connective tissue (the process called fibrosis), leads to thickening of the walls, and causes reduced oxygen supply in the blood. As a consequence patients suffer from perpetual shortness of breath.

The term "pulmonary sarcoidosis" refers to an interstitial lung disease involving abnormal collections of inflammatory cells that form lumps known as granulomas. In pulmonary sarcoidosis, the inflammatory process involves the alveoli, small bronchi and small blood vessels.

In certain preferred embodiments, the inflammatory respiratory disease may be a chronic respiratory disease.

The term "chronic respiratory disease" refers to any chronic disease of the respiratory tract including the airways and other structures of the lung. Chronic respiratory diseases are typically characterized by inflammation.

In certain embodiments, the chronic respiratory disease may be selected from the group consisting of asthma, chronic obstructive pulmonary disease (COPD), chronic bronchitis, and acute respiratory distress syndrome (ARDS).

In certain embodiments, the chronic respiratory disease may be asthma, such as severe (persistent) asthma.

In certain embodiments, the chronic respiratory disease may be COPD.

In certain embodiments, the regulatory macrophages are (suitable) to be administered to an autologous subject.

In certain embodiments, the regulatory macrophages are (suitable) to be administered by intratracheal, intrabronchial, or subcutaneous administration.

In certain embodiments, the regulatory macrophages may be administered by intratracheal or intrabronchial administration.

The term "intratracheal" generally refers to a route of administration in which a pharmaceutically active ingredient (e.g. the regulatory macrophages as taught herein) is delivered into the trachea or windpipe. For example, the delivery can be done by means of injection.

In certain embodiments, the intratracheal administration may be performed by injecting the cells (e.g. in a salt solution) into the trachea or windpipe. The administration may be performed during bronchoscopy, which is routinely performed in clinics.

The term "intrabronchial" generally refers to a route of administration in which a pharmaceutically active ingredient (e.g. the regulatory macrophages as taught herein) is delivered into one or both of the bronchi. The delivery can be done by means of injection.

In certain embodiments, the intrabronchial administration may be performed by injecting the cells (e.g. in a salt solution) into one or both of the bronchi. The administration may be performed during bronchoscopy.

In certain embodiments, the regulatory macrophages may be administered by subcutaneous administration. Advantageously, subcutaneous administration is easier than intratracheal or intrabronchial administration. Further, in certain embodiments wherein the regulatory macrophages as taught herein are cultured prior to the collection step in the presence of one or more antigens, subcutaneous administration may be preferred route of administration. Indeed, given that T cells are circulating in the whole body, induction of regulatory T cells advantageously may be initiated anywhere in the body by the regulatory macrophages as taught herein.

The term "subcutaneous" generally refers to a route of administration in which a pharmaceutically active ingredient (e.g. the regulatory macrophages as taught herein) is delivered or administered into the subcutis, the layer of skin directly below the dermis and epidermis, collectively referred to as the cutis. The delivery can be done by means of injection.

In certain embodiments, the subcutaneous administration may be performed by injecting the cells (e.g. in a salt solution) into the subcutis.

The term "prophylactically effective amount" refers to an amount of an active compound or pharmaceutical active ingredient that inhibits or delays in a subject the onset of a disorder as being sought by a researcher, veterinarian, medical doctor or other clinician. The term "therapeutically effective amount" as used herein, refers to an amount of active compound or pharmaceutical agent that elicits the biological or medicinal response in a subject that is being sought by a researcher, veterinarian, medical doctor or other clinician, which may include *inter alia* alleviation of the symptoms of the disease or condition being treated. Methods are known in the art for determining therapeutically and prophylactically effective doses of the regulatory macrophages or pharmaceutical composition as taught herein.

In the context of the present invention a "therapeutically effective dose" means an amount of the regulatory macrophages or pharmaceutical composition as taught herein that when administered brings about a positive therapeutic response with respect to treatment of a patient with an inflammatory respiratory disease, in particular a chronic respiratory disease, such as asthma or COPD.

Appropriate therapeutically effective doses of the regulatory macrophages or pharmaceutical composition as taught herein may be determined by a qualified physician with due regard to the nature of the regulatory macrophages as taught herein, the disease condition and severity, and the age, size and condition of the patient.

Without limitation, a typical dose of for instance the regulatory macrophages to be administered may range from about 1 x 10⁴ to about 1 x 10¹¹ cells per injection. For example, the dose to be administered may range from about 1 x 10⁵ to about 1 x 10¹⁰ cells per injection, for example, from about 1 x 10⁶ to about 5 x 10⁹ cells per injection. Preferably, the dose to be administered ranges from about 1 x 10⁶ to about 2 x 10⁹ cells per injection.

It is recognized that the treatments of the invention may comprise administration of a single therapeutically effective dose or administration of multiple therapeutically effective doses of the regulatory macrophages or pharmaceutical composition as taught herein.

As used herein, a phrase such as "a subject in need of treatment" includes subjects that would benefit from treatment of a given condition, particularly of an inflammatory respiratory disease, in particular a chronic respiratory disease, such as asthma or COPD. Such subjects may include, without limitation, those that have been diagnosed with said condition, those prone to develop said condition and/or those in whom said condition is to be prevented.

The terms "treating" or "treatment" as used herein refer to both the therapeutic treatment of an already developed disease or condition, as well as prophylactic or preventative measures, wherein the aim is to prevent or lessen the chances of incidence of an undesired affliction, such as to prevent the chances of progression of the disease or condition. Beneficial or desired clinical results may include, without limitation, alleviation of one or more symptoms or one or more biological markers, diminishment of extent of disease, stabilised (i.e., not worsening) state of disease, delay or slowing of disease progression, amelioration or palliation of the disease state, and the like. "Treatment" can also mean prolonging survival as compared with expected survival if not receiving treatment.

A further aspect relates to a pharmaceutical composition comprising the regulatory macrophages as taught herein. Hence, certain embodiments provide a pharmaceutical composition comprising regulatory macrophages, wherein the regulatory macrophages are obtainable or obtained by a method as taught herein. Certain embodiments thus provide a pharmaceutical composition comprising regulatory macrophages, wherein the regulatory macrophages are obtainable or obtained by a method for *in vitro* differentiation of monocytes to regulatory macrophages, the method comprising culturing the monocytes in a medium in the presence of an immunostimulatory nucleic acid, such as a CpG nucleic acid.

In the context of the present invention, the terms "pharmaceutical composition", "pharmaceutical formulation", "formulation", or "composition" may be used interchangeably.

In certain embodiments, the pharmaceutical composition as taught herein may be configured for intratracheal or intrabronchial administration. In certain embodiments, the pharmaceutical composition as taught herein may be administered by intratracheal or intrabronchial injection. Intratracheal or intrabronchial administration of the present pharmaceutical compositions, e.g., by intratracheal or intrabronchial injection, advantageously allows local delivery of the compositions to the respiratory tract, thereby increasing the efficacy of the treatment of an inflammatory respiratory disease, in particular a chronic respiratory disease, such as asthma or COPD.

In certain embodiments, the pharmaceutical composition as taught herein may be configured for subcutaneous administration. In certain embodiments, the pharmaceutical composition as taught herein may be administered by subcutaneous injection. Advantageously, subcutaneous administration allows easier administration of the regulatory macrophages as taught herein compared with intratracheal or intrabronchial administration. Also, subcutaneous administration, e.g., subcutaneous injection, of the regulatory macrophages as taught herein, such as those cultured prior to the collection step in the presence of one or more antigens, may advantageously result in the induction of regulatory T cells.

The pharmaceutical compositions as taught herein may comprise in addition to the herein particularly specified components one or more pharmaceutically acceptable excipients. Suitable pharmaceutical excipients depend on the dosage form and identities of the active ingredients and can be selected by the skilled person (e.g. by reference to the Handbook of Pharmaceutical Excipients 6th Edition 2009, eds. Rowe et al.). As used herein, "carrier" or "excipient" includes any and all solvents, diluents, buffers (such as neutral buffered saline or phosphate buffered saline), solubilisers, colloids, dispersion media, vehicles, fillers, chelating agents (such as EDTA or glutathione), amino acids (such as glycine), proteins, disintegrants, binders, lubricants, wetting agents, emulsifiers, sweeteners, colorants, flavourings, aromatisers, thickeners, agents for achieving a depot effect, coatings, antifungal agents, preservatives, stabilisers, antioxidants, tonicity controlling agents, absorption delaying agents, and the like. The use of such media and agents for pharmaceutical active substances is well known in the art. Such materials should be non-toxic and should not interfere with the activity of the regulatory macrophages as taught herein.

The precise nature of the carrier or other material will depend on the route of administration. For example, the pharmaceutical composition may be in the form of a parenterally (e.g. subcutaneously) acceptable aqueous solution, which is pyrogen-free and has suitable pH, isotonicity and stability.

The pharmaceutical compositions may comprise pharmaceutically acceptable auxiliary substances as required to approximate physiological conditions, such as pH adjusting and buffering agents, preservatives, complexing agents, tonicity adjusting agents, wetting agents and the like, for example, sodium acetate, sodium lactate, sodium phosphate, sodium hydroxide, hydrogen chloride, benzyl alcohol, parabens, EDTA, sodium oleate, sodium chloride, potassium chloride, calcium chloride, sorbitan monolaurate, triethanolamine oleate, etc. Preferably, the pH value of the pharmaceutical composition is in the physiological pH range, such as particularly the pH of the composition is between about 5 and about 9.5, more preferably between about 6 and about 8.5, even more preferably between about 7 and about 7.5. The preparation of such pharmaceutical composition is within the ordinary skill of a person skilled in the art.

As corroborated by the experimental section, which illustrates certain representative embodiments of the invention, in a model of chronic obstructive pulmonary disease the inventors found that an increase in airway hyperreactivity to methacholine was associated with a substantial decrease in the numbers of interstitial macrophages in the lung, and that pretreatment with CpG was able to restore the numbers of interstitial macrophages in the lung to levels observed in non-exposed subjects, which was associated with an improvement in lung function.

Accordingly, a further aspect relates to an immunostimulatory nucleic acid, such as a CpG nucleic acid, for use in the treatment (including throughout the present specification therapeutic and/or preventative measures) of chronic obstructive pulmonary disease (COPD).

A related aspect provides a method of treating chronic obstructive pulmonary disease in a subject in need of such treatment comprising administering to said subject a therapeutically or prophylactically effective amount of an immunostimulatory nucleic acid, such as a CpG nucleic acid.

A related aspect provides the use of an immunostimulatory nucleic acid, such as a CpG nucleic acid, for the manufacture of a medicament for the treatment of chronic obstructive pulmonary disease.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The herein disclosed aspects and embodiments of the invention are further supported by the following non-limiting examples.

### EXAMPLES

### MATERIALS AND METHODS

### Mice

WT CD45.2⁺ C57BL/6 and WT CD45.1⁺ C57BL/6 mice were from the Jackson Laboratory. CD45.1/2⁺ WT C57BL/6 mice were obtained by crossing CD45.1⁺ with CD45.2⁺ mice. *Agtr1a*^{*-*/*-*}, *Ccr2-^{l}-, Cx3cr1*^{*gfp*/*+*}, *Il-10*^{*-*/}*⁻, Myd88*^{*-*/*-*} and *Nr4a1*^{*-*/*-*} mice on the C57BL/6 background were from the Jackson Laboratory. *Tlr9*^{*-*/*-*} were described in Hemmi et al., 2000 (Nature, 408, 740-745) and IL-10-β-lactamase reporter (ITIB) mice were described in Bouabe et al., 2011 (J. Immunol., 187, 3165-3176). All mice, except parabiotic mice that were generated at the Singapore Immunology Network (SIgN, Singapore), were housed and bred in institutional specific pathogen-free facilities at the GIGA-Research center of the University of Liege (Belgium) and were used at 6-10 weeks of age, unless otherwise indicated. All experimental procedures and protocols were reviewed and approved by the Institutional Animal Care and Use Ethics Committee of the University of Liege (Belgium), except for experiments involving parabiotic mice that were approved by the local ethical committee at the Singapore Immunology Network (SIgN, Singapore).

The "Guide for the Care and Use of Laboratory Animals", prepared by the Institute of Laboratory Animal Resources, National Research Council, and published by the National Academy Press, as well as European and local legislations, were followed carefully.

### Reagents and Antibodies

2.4G2 Fc receptor blocking antibodies were produced in house. Fluorogenic coumarin-cephalosporin-fluorescein (4)-acetoxymethyl (CCF4-AM) was from Invitrogen. Pam3CSK4, Poly(I:C), FLA-BS and R848 were from InvivoGen. MALP-2 was from Enzo Life Sciences. LPS from *E*. *coli* O55:B5 was from Sigma. Mouse non-CpG DNA (ODN, 5'-gcttgatgactcagccggaa-3'(SEQ ID NO: 5); Ref. HC4034) and CpG-DNA (5'-tccatgacgttcctgatgct-3'(SEQ ID NO: 6); Ref. HC4033) were from Sanbio. Clodronate-containing liposomes or control liposomes were from Foundation Clodronate Liposomes. 5-ethynyl-2'-deoxy-uridine (EdU) was from Jena Bioscience. HDM extracts (HDM from *Dermatophagoides farinae*) were from Greer Laboratories. OVA grade III and grade V were purchased from Sigma Aldrich. Fluorescent HDM was produced by labelling with the Alexa fluor 647 Protein Labelling kit (Molecular Probes).

PE-conjugated anti-B220 (clone RA3-6B2), APC-, FITC-, PerCP-Cy5.5- and V500-conjugated anti-CD45.2 (clone 104), PE-conjugated anti-CD64 (clone X54-5:7.1), PE-Cy7-conjugated anti-CD11b (clone M1/70), APC- and APC-Cy7-conjugated anti-CD11c (clone HL3), PerCP-Cy5.5-, BV421-conjugated anti-CD49b (clone DX5), FITC-conjugated anti-Ly6C (clone AL-21), PE- and PE-Cy7-conjugated anti-Ly6g (clone 1A8), APC-conjugated anti-MHCII (clone AF6-120.1), PE-conjugated anti-NK1.1 (clone PK136), PE-conjugated anti-SiglecF (clone E50-2440) were purchased from BD Pharmingen. APC-conjugated anti-CD45.1 (clone A20), FITC-conjugated anti-CD11b (clone M1/70), efluor660-conjugated anti-CD169 (clone SER-4), PerCP-Cy5.5-conjugated anti-CD19 (clone 1D3), PE-conjugated anti-CD115 (clone AFS98), efluor 450-conjugated anti-CD3 (clone 17A2), PerCP-Cy5.5-conjugated anti-CD62L (clone MEL-14), biotin-conjugated anti-CD86 (clone GL-1), efluor660-, PerCP-efluor710-conjugated anti-CD90.2 (clone 30H12), PE-conjugated anti-F4/80(clone BM8) were from eBioscience. BV421-conjugated anti-CD64 (clone X54-5:7.1) and PerCP-Cy5.5-conjugated anti-MHCII (clone M5/M4.15.2) were from Biolegend. PE-conjugated anti-CCR2 (clone 475301), and APC-conjugated anti-merTK (clone 108928) were from R&D systems. Anti-CD11b MicroBeads were from Miltenyi Biotec. Appropriate isotype control antibodies were purchased from the same respective manufacturers.

Rabbit anti-phospho-p44/42 MAPK (Erk1/2) and anti-b-Actin (clone D6A8) antibodies were from Cell Signaling Technology. Horseradish peroxidase (HRP)-conjugated goat anti-rabbit IgG antibody was from Kirkegaard & Perry Laboratories. Goat anti-CCL2, rat anti-CCL5 (clone 53405) and rat anti-CCL9 (clone 62105) neutralizing antibodies were from R&D Systems. Appropriate isotype control antibodies were purchased from the same manufacturer.

### Cell isolation, staining and flow cytometry

To obtain single-lung-cell suspensions, lungs were perfused with 10 ml of PBS through the right ventricle, cut into small pieces, and digested for 1 h at 37°C in HBSS containing 1 mg/ml collagenase A (Roche) and 0.05 mg/ml DNase I (Roche). Single-cell suspensions were then enriched in mononuclear cells by harvesting cells from the 1.080:1.038 g/mL interface using a density gradient (Easycoll from VWR). Blood was collected and mixed with EDTA (100 mM), and red blood cells were lysed with RBC lysis buffer (eBioscience). To obtain single-heart and liver-cell suspensions, these organs were cut into small pieces and digested for 1 h at 37°C in HBSS containing 1 mg/ml collagenase A (Roche) and 0.05 mg/ml DNase I (Roche).

Cell phenotyping and sorting were performed on a FACSCANTO II and a FACSARIA III (BD Biosciences), respectively. Staining reactions were performed at 4°C after incubation with 2.4G2 Fc receptor blocking antibodies to reduce non-specific binding.

Regarding cell phenotyping and sorting, FSC-W and FSC-A discrimination was used to exclude doublet cells, and ViaProbe (7-AAD) cell viability solution (BD Biosciences) was used to discriminate between dead and living cells. In experiments aimed at isolating lung Ly-6C^{hi}CD64^{lo} classical monocytes, Ly-6C^{lo}CD64^{lo} patrolling monocytes, AM, IM_{SS}, IM_{CpG}, or splenic monocytes, lung mononuclear cells and splenic cells were first enriched in CD11b-expressing cells by MACS using CD11b microbeads. Results were analyzed with FlowJo software (Tree Star).

### Cytologic examination

Cytologic examination of FACS-sorted populations was performed on cytospin preparations stained with Hemacolor® (Merck KgaA). Sections were examined with a FSX100 microscope (Olympus).

### Vascular monocytes/macrophage depletion

WT or *Ccr2*^{*-*/*-*} mice were injected i.v. with 200 µl of clodronate-containing liposomes (Clo-Lip) or empty liposomes (PBS-Lip), and lung cells were harvested 24 hours later.

### Assessment of IL-10 expression in ITIB mice and IL-10 production ex vivo

To assess IL-10 expression using IL-10-b-lactamase reporter ITIB mice (Bouabe et al., 2011, *supra*), lung cells from IL-10-b-lactamase reporter ITIB and control WT mice were resuspended in a CCF4-AM-containing solution supplemented with probenecid (Invitrogen), prepared according to the manufacturer's instructions, and incubated 90 min at 29°C. Briefly, CCF4-AM is a lipophilic ester derivative that consists of a cephalosporin core bridging a 7-hydroxycoumarin to a fluorescein. Once inside the cell, the ester groups are readily hydrolysed by cytoplasmic esterases, releasing the negatively charged form, CCF4, which is thus trapped in the cytosol (using inhibitor for anion transporters, such as probenicid, further increase trapping substrate/product within the cells). Exciting intact CCF4 at 405 nm induces fluorescence resonance energy transfer (FRET) from the coumarin to the fluorescein that subsequently emits green light. After cleavage of the β-lactam ring of CCF4 by β-lactamase, which is expressed under the control of the *Il10* gene promoter, the two fluorophores are separated, resulting in the disruption of FRET. Excitation at 409 nm now results in blue fluorescence detectable at 450 nm. CCF4-loaded cells were then classically stained and analyzed by flow cytometry. CCF4-loaded WT cells were used as negative controls.

For IL-10 production *ex vivo,* classical monocytes, patrolling monocytes, AM and IM were FACS-sorted from naive WT mice and 2x10⁵ cells were cultured in 200 µl of RPMI medium supplemented with 10% foetal bovine serum (FBS) and additives (L-glutamine 8mM, MEAA 0.1 mM, Pyruvate Na 1mM, Penicillin/streptomycin 50UI/ml, β-mercaptoethanol 0.5mM) in a 96 well plate during 16 h. In some experiments, IM isolated from WT, *Tlr9*^{*-*/*-*} or *Myd88*^{*-*/*-*} mice were cultured and stimulated during 16h with LPS (10 ng/ml) or CpG (7 µg/ml). Concentrations of IL-10 were measured by ELISA (eBioscience) in culture supernatants.

### Quantification of monocytes/macrophages numbers

Lung cell numbers were counted following whole lung digestion and mononuclear cell enrichment by the use of a hemocytometer. The numbers of monocytes/macrophages were determined according to the gating strategy described in Example 1.

Total BALF cell numbers were counted by the use of a hemacytometer. The numbers of IMₛₛ and IM_{CpG} were determined according to the gating strategy described in Example 1.

Total splenic, cardiac, liver and bone marrow cell numbers were counted following tissue dissociation by the use of a hemacytometer. The numbers of splenic, cardiac, liver and bone marrow monocytes or macrophages were determined according to the gating strategy described by Swirski and collaborators (Swirski et al., 2009, Science, 325, 612-616) or described in Example 1, respectively.

### Immunohistochemistry

Lungs were fixed in 4% formalin, paraffin-embedded and cut in 5 µm thick sections. Deparaffinized tissues were dewaxed and endogenous peroxides were blocked with hydrogen peroxide 3% (Merck). Tissue sections were then immersed in protein block serum free (Dako) for 10 min at room temperature. Mouse lung sections were stained with a rat anti-mouse Ly-6C (dilution 1/50) for 2 h at room temperature (Abcam) followed by a HRP-coupled rabbit anti-rat staining (dilution 1/400) for 40 min at room temperature (Abcam). Peroxidase activity was revealed using the 3,3'-diaminobenzidine hydrochloride kit (Dako), and tissues were then counter-colored with Carazzi's hematoxylin. Slides were mounted on Eukitt medium (Fluka Analytical).

### In vivo labeling of vascular leukocytes

WT mice were injected i.v. with 0.5 µg of anti-CD45.2 antibodies. The antibody was allowed to circulate for 3 minutes prior to euthanasia in order to label all leukocytes in the vascular space. Lungs were then harvested without being perfused and the monocyte/macrophage populations were analyzed by flow cytometry.

### Airway exposure to TLR ligands and pathogens

Lightly isoflurane anesthetized mice were instilled i.n. with 50 µl saline or 50 µl saline containing various TLR ligands (i.e. Pam3CSK4 25 µg; Poly(I:C) 50 µg; LPS 10 µg; FLA-BS 1 µg; MALP-2 1 µg; CpG-DNA 50 µg) or respiratory pathogens (i.e. influenza A 5 PFU; *S. pneumoniae* serotype 1, clinical isolate E1586, 105 CFU). The doses of TLR ligands used were chosen according to the ones found in the literature: Pam3CSK4 (Andrade et al., 2013, J. Immunol., 191, 4759-4768; Chen et al., 2016, PloS One, 11, e0149233); Poly(I :C) (Errea et al., 2015, Immunol. Lett., 164, 33-39; LeMessurier et al., 2013, PLoS Pathog. 9, e1003727); LPS (Chen et al., 2014, PloS One, 9, e101925; Hiroshima et al., 2014, J. Immunol., 192, 2800-2811); FLA-BS (Errea et al., 2015, *supra;* Munoz et al., 2010, Infect. Immun., 78, 4226-4233); MALP-2 (Reppe et al., 2015, Infect. Immun., 83, 4617-4629); R848 (Marino et al., 2011, J. Immunol., 186, 4433-4442; Van et al., 2011, Eur. J Immunol., 41, 1992-1999); CpG (Hickey et al., 2013, Infect. Immun., 81, 2123-2132; Vogel and Brown, 2015, Front. Immunol., 6, 327). Influenza type A virus strain A/PR/8/34 (PR8) was provided by Dr François Trottein (Institut Pasteur de Lille, France).

### House dust mite-induced model of asthma and CpG treatments

Lightly isoflurane anesthetized WT, ITIB or *Tlr9*^{*-*/*-*} mice were sensitized by two weekly i.n. instillations of vehicle (LPS-free saline) or HDM extracts (100 µg in 50 µl) on day 0 and on day 7.

For assessing the effects of CpG in preventing asthma development (Figure 3), WT mice were transferred i.t. in adoptive transfer experiments with 1.5x10⁶ AM or IM_{CpG} sorted from CpG-treated WT or *Il-10*^{*-*/*-*} mice (mortality rate varying between 40 and 60% in CpG-injected *Il-10*^{*-*/*-*} mice, but not WT mice, unpublished observation), or vehicle (50 µl saline), 24 hours before each HDM treatment (days -1 and 6).

For assessing the curative effects of CpG on established asthma (Figure 4), WT mice were transferred i.t. in adoptive transfer experiments with 1.5x10⁶ AM or IM_{CpG} sorted from CpG-treated WT or *Il-10*^{*-*/*-*} mice (mortality rate varying between 40 and 60% in CpG-injected *Il-10* mice, but not WT mice, unpublished observation), or vehicle (50 µl saline), 24 hours before the last HDM challenge (day 20). In experiments assessing the long-lasting effects of CpG on established asthma, mice were challenged with HDM 8 weeks after a single CpG instillation and were sacrificed 3 days later.

### Assessment of the asthmatic response in HDM-exposed mice

AHR was estimated by assessing dynamic airway resistance in anesthetized animals subjected to increasing doses of methacholine using a FlexiVent small animal ventilator (SCIREQ), as previously described (Gueders et al., 2009, Inflamm. Res., 58, 845-854). Mice were sacrificed and BALF, lungs and lymph node cells were harvested and analyzed as described previously (Marichal et al., 2010, J. Allergy Clin. Immunol., 126, 836-844). Briefly, BALF eosinophilia was evaluated by total and differential cell counts, lung inflammation and mucus production were evaluated on H&E- and PAS-stained lung sections, respectively. Mucus production was also quantified as the percentage of PAS-stained goblet cells per total epithelial cells in randomly selected bronchi. Seven randomly selected sections were analyzed per murine lung. Bronchial lymph node cells were cultured *in vitro* in supplemented Click's medium (10% FBS, L-glutamine 8mM, modified essential amino acids 0.1 mM, sodium pyruvate 1mM, Penicillin/Streptomycin 50 UI/ml and β-mercaptoethanol 0.5mM) with or without HDM restimulation (30 µg/ml). Culture supernatants were assayed for cytokine production (IL-4, IL-5, IL-13) by ELISA (eBioscience) after 3 days of culture.

### Generation of bone marrow mixed chimeras and parabiotic mice

Twelve week-old CD45.1/2+ WT C57BL/6 mice were lethally irradiated with two doses of 6 Gy 3 hours apart. Two hours after the second irradiation, these mice were injected i.v. with 2x10⁶ BM cells consisting of a 1:1 mix of BM cells obtained from CD45.1⁺ C57BL/6 and CD45.2⁺ *Ccr2*^{*-*/*-*} C57BL/6 mice. Mice were treated with antibiotics (Baytril, Bayer, 0.05 mg/ml in drinking water) from the day before irradiation until the time of sacrifice. Cell numbers were evaluated 7, 14, 56 and 84 days later and chimerism was assessed 56 and 84 days later. In some experiments, such BM mixed chimeras were instilled with CpG or LPS 7 days before the analysis.

Parabiotic mice were generated by suturing age- and weight-matched WT CD45.1⁺ and *Ccr2*^{*-*/*-*} CD45.2⁺ mice at the age of 6 weeks. Mice were analyzed at 6 months of age.

### Assessment of cell proliferation using EdU incorporation

One, two or three days after i.n. administration of CpG, C57BL/6 WT or *Ccr2*^{*-*/*-*} mice were injected i.p. with EdU (1 mg in 400 µl). Mice were sacrificed 24 hours later and incorporation of EdU was assessed by flow cytometry.

### Ex vivo stimulation of tissue monocytes with CpG and LPS

Lung and splenic monocytes were sorted by FACS according to the gating strategy described in Example 1 and by Swirski et al. (2009, *supra*), respectively. Two hundred thousand (2x10⁵) cells were cultured in RPMI medium supplemented with 10% FBS and additives (L-glutamine 8 mM, MEAA 0.1 mM, Pyruvate Na 1mM, Penicillin/streptomycin 50UI ml-1, β-mercaptoethanol 0.5 mM), with or without CpG (7 µg ml-1) or LPS (10 ng ml-1) in a 24 well plate. Twenty-four, 36, 48 and 54 hours later, cells were harvested and phenotyped. ViaProbe (7-AAD) cell viability solution was used to discriminate between dead and living cells.

### Western blot

Five hundred thousand lung monocytes were sorted by FACS from naive *Ccr2^{-l-}* mice and were cultured in RPMI medium supplemented with 10% FBS and additives (L-glutamine 8mM, MEAA 0.1 mM, Pyruvate Na 1mM, Penicillin/streptomycin 50UI/ml, β-mercaptoethanol 0.5mM) in a 12-well plate. Cells were treated with or without oligodeoxynucleotide (ODN) or CpG-DNA (7 µg/mL) for 30 minutes. Cells were then harvested, centrifuged and pellets were lysed in radioimmunoprecipitation assay (RIPA) lysis buffer with 1 mM Na3VO4, 10 mM NaF and complete protease inhibitor (Roche Diagnostics). Protein extracts were added to Laemmli buffer, boiled and run on a 10% polyacrylamide-SDS gel; after electrophoresis, gels were electro-transferred onto a polyvinylidene difluoride membrane (PVDF) for western-blotting. Nonspecific binding was blocked in 20 mM Tris, pH 7.5, 500 mM NaCl, 0.2% Tween 20 (TBS-Tween), and 5% dry milk during 1 h. Membranes were then incubated with rabbit antiphospho-p44/42 MAPK (Erk1/2), anti-p44/42 MAPK (Erk1/2) or anti-b-Actin antibodies (1:1,000; Cell Signaling Technology) overnight at 4°C, rinsed with TBS-Tween and further incubated with horseradish peroxidase (HRP)-conjugated goat anti-rabbit IgG (1:5,000; Kirkegaard & Perry Laboratories) for 60 min at room temperature. Result was visualized using an enhanced chemiluminescence system (ECL kit; Amersham) followed by autoradiography with Fuji x-ray films.

### mRNA isolation, microarray and Gene Set Enrichment Analyses

Singlet living Ly-6C^{hi}CD64^{lo} cells from *Ccr2*^{*-*/*-*} or WT mice, and AM, IM_{SS} and IM_{CpG} from WT mice were directly sorted in Trizol® (Life Technologies). The 4 cell populations were sorted in duplicate (IM_{CpG}) or triplicate (all the others), yielding a total of 11 samples. Total RNA was purified using Direct-zol® kit (Zymo Research) following manufacturer's recommendations. For each cell type, total RNAs were eluted in a total of 175 µl of water. Eluted RNAs were incubated with DNase for 10 min at room temperature in a total volume of 200 µl using the RNase-Free DNase Set from Qiagen (Venlo), followed by purification on RNeasy® Micro columns (Qiagen) and final elution in 50µl water. RNAs were quantitatively and qualitatively verified using Agilent RNA 6000 Pico Kit run on an Agilent 2100 Bioanalyzer. Concentrations were around 1ng/µl and RNA Integrity Numbers were above 8. 1.5ng RNA was used to generate fragmented and biotin-labeled ssDNA targets using GeneChip® WT Pico Reagent Kit (Affymetrix). The fragmented and labeled targets were hybridized to GeneChip® Mouse Gene 1.0 ST Arrays following standard Affymetrix protocol, which includes overnight hybridization at 45°C and 60 rpm in an Affymetrix GeneChip Hybridization Oven 645. The arrays were then washed and stained in an Affymetrix GeneChip Fluidics Station 450.

Microarrays were analyzed using R (version 3.3.1) and Bioconductor (Huber et al., 2015, Nat. Methods, 12, 115-121). Raw data were background-corrected and quantile-normalized using "Robust Multichip Average algorithm" (RMA) from "oligo" package. Normalized data were then *a*nnotated using "mogene10sttranscriptcluster.db" annotation package.

Differential expression analysis between Ly-6C^{hi} monocytes from *Ccr2*^{*-*/*-*} mice and Ly-6C^{hi} monocytes from WT mice was performed using "Linear Models for Microarray Data" (Limma) package in Bioconductor, with thresholds as following: Benjamini Hochberg adjusted *P* value (adj *P* value) < 0.05 AND Fold Change (FC) > 2 or < 0.5, and after having filtered out non annotated probes. Differential expression analysis between IMₛₛ and IM_{CpG} was performed identically, excepted for adj *P* value threshold set to 0.05. Volcano plot of differences between Ly-6C^{hi} monocytes from *Ccr2*^{*-*/*-*} mice and Ly-6C^{hi} monocytes from WT mice was obtained by plotting log₂ (FC) on the x axis and -log₁₀ (adj *P* value) on the y axis for all probes present in the Affymetrix GeneChip® Mouse Gene 1.0 ST Array, including non-annotated ones.

Heatmaps were generated by plotting normalized expression values of relevant samples and genes using "heatmap.2" function from "gplots" package.

Principal Component Analysis (PCA) was performed using "prcomp" function on normalized expression values of AM, IMₛₛ and IM_{CpG}. Values of principal components 1, 2 and 3 were then tridimensionnally plotted using "plot3d" function of "rgl" package. Percentages shown on each axis indicate the % of variability explained, with PC1 explaining the most variability.

In order to identify biological signatures of IM_{CpG} compared to IMₛₛ, it was decided to employ the commonly used non-parametric, rank based tool, Gene Set Enrichment Analysis (GSEA version 2.2.2) (Subramanian et al., 2005, P. Natl. Acad. Sci. U.S.A, 102, 15545-15550). Microarray normalized expression data of IMss (3 samples) and IMCpG (2 samples) were used by GSEA as dataset to rank genes between the two cell types. Since (i) IM_{CpG} class contains 2 samples and (ii) RMA normalized data are in log scale, «Diff_of_Classes» method was chosen to rank genes in the dataset. Enrichment analysis was then performed using hallmark gene sets of MsigDB database.

### Real-time PCR analysis

Singlet living lung Ly-6C^{hi} cells from *Ccr2*^{*-*/*-*} mice, and lung IM, AM and splenic monocytes from WT mice were directly sorted in Trizol® (Life Technologies). Total RNA was isolated using Direct-zol® kit (Zymo Research) following manufacturer's recommendations. Complementary DNA was obtained using RevertAid First Strand cDNA Synthesis Kit (Thermo Fisher Scientific). Quantitative real-time PCR were performed in duplicates using ABsolute Blue qPCR SYBR Green ROX Mix (Thermo Fisher Scientific) and ABI 7900HT Fast Real-Time PCR System (Applied Biosystems) under the following conditions: (1) initial denaturation at 95°C for 15 min followed by (2) 40 cycles of 95°C 15 s and 60°C 1 min. Primer sequences were as followed :. 5'-AGCTGAACATGAACGGCATCT-3' (SEQ ID NO: 7) and 5'-GATCGGCCAGCCATCTGA-3' (SEQ ID NO: 8) for *Tlr9,* 5'-AGCCCAGTGTTACCACCAAG-3' (SEQ ID NO: 9) and 5'-ACCCAAGAACAAGCACAAGG-3' (SEQ ID NO: 10) for the housekeeping gene *Ubc, 5'-*CATGGCTCGCTCGGTGACC-3' (SEQ ID NO: 11) and 5'-AATGTGAGGCGGGTGGAACTG-3' (SEQ ID NO: 12) for the housekeeping gene *B2m,* 5'-AGCTACTGTAATGATCAGTCAACG-3' (SEQ ID NO: 13) and 5'-AGAGGTCCTTTTCACCAGCA-3' (SEQ ID NO: 14) for the housekeeping gene *Hprt.* Expression levels of TLR9 were normalized relative to three control genes (*Ubc, B2m* and *Hprt)* using qBase+ software (Biogazelle).

### s.c. and i.p. CpG treatments

WT mice were injected s.c. or i.p. with 100 µl saline or 200 µl (i.p.) or 100 µl (s.c.) saline containing 50 µg CpG. Mice were analyzed 7 days later.

### Splenectomy

*Ccr2*^{*-*/*-*} mice were anesthetized by intraperitoneal injection of ketamine (75 mg kg-1), xylazine (10 mg/kg) and buprenorphine (0.05 mg/kg). A transversal incision was made on the left lower back of the mice to expose the spleen. Spleen was resected and vessels were cauterized.

The incision was sutured. Sham-operated mice underwent the same procedure without removal of the spleen.

### Chemokine array

Lightly isoflurane anesthetized WT mice were instilled i.n. with 50 µl saline or 50 µl saline containing either CpG (50 µg) or LPS (10 µg). Eight hours later, mice were sacrificed and lungs were harvested without being perfused. Lungs were homogenized with an UltraTurrax homogenizer (IKA) in 2 ml saline containing complete protease inhibitor (Roche Diagnostics). Samples were then centrifuged for 10 minutes at 16,000 x g and supernatants were tested for the presence of chemokines using a chemokine antibody array (Abcam) according to manufacturer instructions. Membranes were developed and digitized for analysis using ImageJ. Each sample spot density was measured, averaged and normalized to membrane reference spots.

### Chemokine inhibition

*Ccr2*^{*-*/*-*} mice were injected i.p. with neutralizing anti-CCL2 (200 µg), anti-CCL5 (50 µg), anti-CCL9 (50 µg) antibodies or a combination of these 3 antibodies, 30 minutes before an i.n. administration of CpG. Control mice received the appropriate isotype controls. The numbers of lung monocytes and IM_{CpG} were analyzed 1 day and 7 days after CpG administration, respectively.

### Statistical Analysis

Statistical analyses were performed using R (version 3.2.4). Data from independent experiments were pooled for analysis in each data panel, unless otherwise indicated. Raw data was transformed when needed and was back-transformed for graphical presentation. Respect of the assumptions of normal distribution of residuals and homoscedasticity was verified using diagnostic plots. We considered a *P*-value lower than 0.05 as significant. *, *P*<0.05; **, *P*<0.01, ***, *P*<0.001; ns, not significant.

### Example 1: Bacterial CpG-DNA prevents asthma by expanding lung interstitial regulatory macrophages from local and splenic reservoir monocytes

### A Simplified Flow Cytometry Strategy Discriminates Lung Monocyte/Macrophage Subsets in Mice

In order to identify the monocyte/macrophage populations associated to the lung at steady state, the inventors set up a 6-step 5-color flow cytometry staining on mononuclear cell enriched lung cells isolated from whole, non-lavaged, perfused lungs of naive C57BL/6 WT mice. To encompass all monocyte/macrophage populations, we gated sequentially on living singlet cells (Step 1), CD45⁺ immune cells (Step 2) and cells positive for the pan monocyte/macrophage marker F4/80 (Step 3). Alveolar macrophages (AM), the most abundant lung phagocytic cells present in the airways, were identified as large (FSC^{hi}) autofluorescent CD11c^{hi}SiglecF^{hi}CD64⁺Merk⁺ CD169⁺ cells (Step 4). F4/80-expressing lung-resident eosinophils were excluded based on their high granularity (SSC^{hi}) (Step 5). The remaining CD45⁺F4/80⁺ cells were further separated into three populations according to their expression of the monocyte and macrophage markers Ly-6C and CD64, respectively (Step 6).

Ly-6C^{hi}CD64^{lo} cells were classical monocytes, as they exhibited a monocytic morphology, expressed the typical markers of classical monocytes (Ly-6C^{hi}CCR2⁺CX3CR1^{int}CD62L⁺) and were less abundant in *Ccr2*^{*-*/*-*} mice (Figure 1A), in which classical monocyte emigration from the bone marrow (BM) is compromised.

Ly-6C^{lo}CD64^{lo} cells also displayed a monocytic morphology, expressed the characteristic markers of patrolling monocytes (Ly-6C^{lo}CCR2^{lo}CX3CR1^{hi}CD62L⁻) and were significantly reduced in mice lacking NR4A1 (Figure 1A), a transcription factor controlling differentiation and survival of patrolling monocytes, demonstrating that these cells were patrolling monocytes.

Finally, Ly-6C^{lo}CD64^{hi} cells were identified as interstitial macrophages (IM). Indeed, these cells were absent in the broncho-alveolar lavage fluid (BALF) and were positive for MHC-II and the macrophage markers Mertk and CD 169. IM were already present after birth, were found in similar numbers in 6-10 week-old *Ccr2*^{*-*/}*⁻, Nr4a1*^{*-*/*-*} and WT mice (Figure 1A), but their numbers were significantly reduced in 1-year-old *Ccr2*^{*-*/*-*} mice as compared to age-matched controls (results not shown), suggesting that IM are partially maintained by classical monocytes in aged adults and might represent a heterogeneous population. Moreover, they constitutively expressed IL-10, as quantified *in vivo* by the use of IL-10-β-lactamase reporter ITIB mice (Figure 1B) and *ex vivo* by ELISA (Figure 1C). Notably, spontaneous IM-derived IL-10 production was dependent on the adaptor molecule MYD88, but independent of TLR9, concordant with the fact that LPS, which mediates signaling through MYD88, sustains IM-derived IL-10 at steady-state. Upon CpG or LPS stimulation, the increased ability of IM to produce IL-10 relied, respectively, on TLR9 and MyD88, or MyD88 only.

When applied on lungs during house dust mite (HDM)-induced airway allergy, the flow cytometry strategy identified similar cell populations.

To determine which cells were truly located in the lung tissue, we depleted blood vessel-associated monocytes/macrophages by injecting clodronate-containing liposomes (Clo-Lip) i.v. 24 hours before analysis (Figure 1D). Such regimen had no effect on AM and IM but efficiently depleted patrolling monocytes and a substantial part (>60%) of Ly-6C^{hi} monocytes (Figure 1E), showing that most monocytes, except some Ly-6C^{hi} monocytes, are associated to the lung vasculature. Concordant with this, combined *in vivo* and *ex vivo* anti-CD45 stainings confirmed that AM, IM and only a fraction of Ly-6C^{hi} monocytes were truly located in the lung of WT mice. Anti-Ly-6C immunostaining of lung sections from Clo-Lip-treated WT and *Ccr2*^{*-*/} mice confirmed that the Ly-6C⁺ cells were located in the lung parenchyma. Notably, in *Ccr2*^{*-*/*-*} mice, Clo-Lip injection did not affect the amount of Ly-6C^{hi} monocytes, whose numbers and localization were similar to those from Clo-Lip treated WT mice (Figure 1E), indicating that such monocytes establish in the lung independently of CCR2. Herein, these lung-resident CCR2-independent monocytes, which are already present at the age of three days, are referred as "lung monocytes". To assess whether lung monocytes represented a distinct population of monocytes compared to classical CCR2-dependent blood monocytes, we performed a transcriptomic comparison of Ly-6C^{hi} monocytes recovered from perfused lung of WT mice (>60% of CCR2-dependent classical blood monocytes and >40% of lung monocytes) and *Ccr2*^{*-*/*-*} mice (more than 95% of lung monocytes). This analysis revealed only 1 differentially expressed gene, namely *Ccr2* (Figure 1F), showing that lung monocytes fully correspond to classical Ly-6C^{hi} monocytes.

### CpG induces TLR9-dependent expansion of IM

The present inventors set out to test whether exposure to certain PAMPs, such as LPS or CpG-DNA (CpG), protects against asthma by enhancing the immunosuppressive potential of IM. First, the inventors confirmed expression of most TLRs on IM and assessed how a local exposure to PAMPs, especially TLR ligands, or a local infection, would quantitatively affect IM. Although several PAMPs, including LPS, were able to significantly expand IM, CpG was by far the most potent in increasing IM numbers one week after a single intranasal (i.n.) exposure (42.5-fold with an average of 1.7 x 10⁶ cells) (Figure 2A, left panel), a phenomenon that was dependent on signaling through TLR9 (Figure 2B). Such expansion persisted up to 8 weeks post-treatment, was also observed to a lesser extent when CpG were administered by a systemic route, and was associated with the presence of a small fraction of CpG-induced IM (IM_{CpG}) into the broncho-alveolar lavage fluid. Conversely, pulmonary infection with *Influenza A* (PR8) or *Streptococcus (S.) pneumoniae* did not have any significant effect on IM numbers 7 days after infection (Figure 2A, right panel). Of note, AM counts remained unchanged post CpG and were moderately increased post LPS.

The inventors compared gene expression profiles between IM_{CpG} and steady-state IM (IMₛₛ). Principal component analysis (PCA) revealed that IM_{CpG} clustered most closely with IMₛₛ compared to AM (Figure 2C), but 285 genes were differentially regulated between IM_{CpG} and IMₛₛ (FC>2, *P*<0.01). Gene set enrichment analysis (GSEA) revealed a significant enrichment, among genes upregulated in IM_{CpG}, of genes implicated in macrophage activation and differentiation, such as targets of c-MYC or genes upregulated by activation of mTORC1 or NOTCH signaling (results not shown).

Protein expression of several surface markers was comparable between IM_{CpG} and IMₛₛ, except for CD11b and CD115, as well as for CD62L, SiglecF and CD169 which were up- and downregulated, respectively. Moreover, CX3CR1, SiglecF and MHC-II expression profiles suggested some degree of heterogeneity within IM_{CpG} (results not shown).

Functionally, IM_{CpG} exhibited a hypersuppressive profile as compared to IMₛₛ. Indeed, the percentage of IL-10⁺ cells within IM_{CpG} was increased, leading to a 55-fold increase in the numbers of IL-10-producing IM upon CpG treatment (Figure 2D). Furthermore, the level of IL-10 expression within IM_{CpG} was substantially greater as compared to the one of IMₛₛ (Figure 2D), leading to a global 550-fold increase in the amount of IM-derived IL-10 expressed in the lungs following CpG treatment. LPS stimulation did not promote such enhanced suppressive profile (Figure 2D).

Altogether, the data show that IM specifically expand in response to CpG exposure and that CpG-induced interstitial macrophages (IM_{CpG}) acquire distinct transcriptomic, phenotypic and functional features.

### CpG-induced IM prevent allergic sensitization to inhaled allergens

The inventors investigated whether the hyper-suppressive profile of IM_{CpG} may contribute to the protective effects of CpG on asthmatic sensitization. To test whether IM_{CpG} sorted from lungs of CpG-treated WT mice were sufficient to confer such protection, adoptive transfer experiments were performed (Figure 3A). Airway hyperreactivity (AHR), eosinophilia, house dust mite (HDM)-specific Th2 responses, lung inflammation and mucus production were significantly reduced when IM_{CpG} sorted from lungs of CpG-treated WT mice were transferred before HDM exposures (Figures 3B to 3E). Such protection was specific for IM_{CpG} and relied on IM_{CpG}-derived IL-10, as transfer of WT AM or *Il-10*^{*-*/*-*} IM_{CpG} failed to improve the characteristic features of asthma (Figures 3A to 3E).

These results demonstrate that IL-10-producing interstitial macrophages (IM_{CpG}) are essential for the protective effects of CpG against sensitization to inhaled allergens.

### CpG-induced IM mediate the immunotherapeutic effects of CpG on established asthma

Since CpG has already proven its efficacy in attenuating established asthma in animal models and in human clinical trials, the inventors set out to develop a model of immunotherapy based on adoptive transfer of IM_{CpG} before HDM challenge in HDM-sensitized mice (Figure 4A). IM_{CpG} were sufficient to confer protection, as adoptive transfer of IM_{CpG} before HDM challenge markedly decreased airway allergy in HDM-sensitized mice, an effect that was not observed when WT AM or *Il10^{-l-}* IM_{CpG} were transferred (Figures 4A to 4E). Importantly, the expansion of IL-10-producing IM and the immunotherapeutic effects of CpG persisted up to 8 weeks post CpG. Moreover, a similar CpG-induced IM-mediated protection was observed in a model of asthma based on systemic sensitization with ovalbumin and alum (results not shown).

### CpG expand IM from CCR2-independent monocytes

Given the crucial contribution of IM_{CpG} to asthma prevention and treatment as illustrated herein, the inventors sought to determine the origin of these regulatory macrophages. In adult tissues, macrophage are maintained or expanded by local proliferation or differentiation from CCR2-dependent bone marrow (BM) derived classical monocytes.

First, to assess the proliferative ability of IM, the incorporation of EdU by IM following CpG treatment was analyzed. CpG exposure did not increase the percentage of EdU+ IM at the time points analyzed, providing no support for local proliferation of IM post CpG (results not shown).

Second, the inventors quantified the numbers of IM_{CpG} in *Ccr2*-/- mice. Surprisingly, IM_{CpG} were found in similar numbers in WT and *Ccr2-*/*-* mice (Figure 5A), while IM_{LPS} numbers were significantly reduced in *Ccr2-*/*-* mice, supporting the idea that IM_{CpG}, unlike IM_{LPS}, develop from monocytes that are recruited independently of the bone marrow. To test this, the inventors used mixed BM competitive CD45.1/2 chimeras engrafted with CD45.1⁺ WT and CD45.2⁺*Ccr2*^{*-*/*-*} BM cells (Figure 5B). Eight weeks after reconstitution, >85% of blood Ly-6C^{hi} monocytes were of CD45.1⁺ WT origin, whereas B lymphocytes and neutrophils had a ratio of 1:1 of CD45.1⁺ WT:CD45.2⁺ *Ccr2*^{*-*/*-*} cells (Figure 5C). Eight and 12 weeks after reconstitution, lung AM and IM were found in comparable numbers as before irradiation and originated mainly from CD45.1+ WT donor cells, showing that the depleted niches were repopulated by CCR2-dependent classical monocytes after irradiation (Figure 5C). Seven days after CpG treatment, BM-competitive chimeras exhibited increased IM numbers and such expansion was associated with a drastic enrichment in CD45.2⁺ *Ccr2*^{*-*/*-*} donor cells, to a level observed in blood B lymphocytes, namely 100% of CCR2 independency when mice were analyzed at 12 weeks of age (Figure 5C). These results demonstrate that IM_{CpG} arise from CCR2-independent precursors. Notably, when chimeric mice were exposed to LPS, the chimerism of IM was not substantially affected and was similar to that of blood Ly-6C^{hi} monocytes, demonstrating that IM_{LPS} arise from BM derived classical monocytes.

Altogether, these results support that interstitial macrophages neither expand through local proliferation nor substantially differentiate from CCR2-dependent BM-derived classical monocytes, but originate from CCR2-independent cells.

### Lung and splenic monocytes are the precursors of IM_{CpG}

Earlier herein, lung monocytes were defined as classical monocytes residing in the lung parenchyma. Given that IM expansion upon CpG sensing was mainly independent of CCR2, it was hypothesized that lung monocytes could act as a local reservoir for IM_{CpG}.

First, the inventors validated the CCR2 independency of lung monocytes by conducting parabiosis and BM mixed chimeras experiments.

CD45.1⁺ WT and CD45.2⁺ *Ccr2*^{*-*/*-*} mice were sutured together for 6 months, and the enrichment in WT and *Ccr2*^{*-*/*-*} partner cells within the *Ccr2*^{*-*/*-*} and the WT hosts, respectively, were analyzed 24 hours after i.v. Clo-Lip treatment (Figure 6A). Parabiotic mice exchanged efficiently their circulation, as attested by the % of enrichment in blood B cells that reached 50%. Conversely, as CCR2 is required for the egress of classical monocytes from the BM, CD45.1⁺ WT monocytes constituted more than 90% of the monocytes present in the blood of both partners. Lung AM were not enriched in partner circulating cells, confirming that AM can self-renew by local proliferation at steady-state (Figure 6A). Regarding lung monocytes, a comparable enrichment in partner cells was observed in WT and *Ccr2*^{*-*/*-*} hosts (Figure 6A), indicating a CCR2-independent turnover of these cells. In BM-reconstituted competitive chimeras, lung monocytes were also repopulated with CCR2-independent engrafted cells, consistent with our findings in parabiotic mice (Figure 6B).

The data so far are consistent with the idea that a precursor-product relationship could exist between lung monocytes and IM_{CpG}. When analyzing the kinetics of appearance of IM_{CpG} *in vivo,* a differentiation pattern from Ly-6C^{hi}CD64^{lo} to Ly-6C^{lo}CD64^{hi} cells was observed, both in WT and *Ccr2*^{*-*/*-*} mice. The appearance of the Ly-6C/CD64 plot looked like a "waterfall", with the progressive acquisition of CD64 and the subsequent loss of Ly-6C, supporting that lung monocytes might give rise to IM_{CpG} (results not shown).

Next, the inventors tested whether lung monocytes could respond to CpG and differentiate into IM-like cells *ex vivo.* FACS-sorted lung monocytes expressed high levels of *Tlr9* as compared to AM or IM and were able to respond to CpG, as shown by increased phosphorylation of the Erk kinase. *Ex vivo* CpG treatment of lung monocytes promoted their survival and triggered the same "waterfall" over time as *in vivo* (Figure 6C), a phenomenon that was dependent on TLR9. Of note, such waterfall was not observed in LPS-stimulated lung monocytes. The data thus support that lung monocytes can differentiate into IM_{CpG}. Nevertheless, the quantity of lung Ly-6C^{hi} cells and IM_{CpG} 1 and 7 days after CpG treatment, respectively (Figure 6D), largely exceeded the number of lung monocytes present at steady-state (Figure 1E). The inventors thus tested whether such lung monocytes were able to proliferate *in situ,* but no increase in EdU incorporation was detected 1 day after CpG, when their numbers were increased >10-fold as compared to day 0, both in WT and *Ccr2*^{*-*/*-*} mice (Figure 6D). Of note, after LPS treatment, such increase in the numbers of Ly-6C^{hi} cells was only seen in WT, but not in *Ccr2*^{*-*/*-*} mice, adding further support to BM origin of IM_{LPS}. These results suggest that, specifically upon CpG stimulation, additional CCR2-independent monocytes are recruited from a distal reservoir and differentiate into IM_{CpG}.

Interestingly, the spleen has been shown to constitute a reservoir of CCR2-independent monocytes, which are already present in 3-day-old mice, and which can migrate to inflammatory tissues in a angiotensin type 1 receptor (AT-1)-dependent manner. Splenic monocytes expressed high levels of *Tlr9* compared to AMs, and, like for lung monocytes, *ex vivo* CpG stimulation promoted their survival and differentiation into IM_{CpG}-like cells (Figure 7A), showing that they can respond to CpG. Notably, 1 day after CpG i.n. treatment, splenic monocyte numbers were significantly decreased, which was associated with an increase in Ly-6C^{hi} monocytes in the lung, both in WT mice and in mice lacking AT-1 receptor (*Agtr1a-*/*-*) (Figure 7B). To assess the exact contribution of splenic monocytes to the pool of lung monocytes and IM_{CpG}, the inventors quantified their numbers 1 and 7 days post CpG, respectively, in splenectomized *Ccr2*^{*-*/*-*} mice. As shown in Figure 7C, the pools of lung monocytes and IM_{CpG} were significantly reduced when the spleen was absent. IMₛₛ numbers were unaffected by splenectomy. Thus, the spleen constitutes a reservoir of monocytes that can seed the lung independently of angiotensin signaling and differentiate into IM_{CpG}.

Of note, when injected s.c. in *Ccr2*^{*-*/*-*} mice, CpG induced recruitment of monocytes not only in the lung, but also in other organs such as the heart or liver. However, except in the lung, this was not associated with an increase in macrophage numbers 7 days later. Conversely, CpG injected i.n. in *Ccr2*^{*-*/*-*} mice promoted recruitment of monocytes in the lung, but not in the heart or liver (data not shown). Altogether, these data suggest that CpG can mobilize CCR2-independent monocytes, but that additional lung-specific signals direct their seeding into the lung and their differentiation into regulatory IM.

In an attempt to identify the chemokines involved in splenic monocyte recruitment, the inventors performed a chemokine array on lung extracts and found that CpG instillation was inducing increased levels of several chemokines, an effect that was however also observed with LPS (Figure 7D). Among those chemokines, CCL2 (MCP-1), CCL5 (RANTES) and CCL9 (MIP-1g) were not only the most prominent ones but also the ones whose respective receptors (i.e., CCR2, CCR5 and CCR1) were the most expressed in Ly-6C^{hi} monocytes (Figure 7D). To assess the potential role of these chemokines, neutralizing antibodies were used *in vivo.* Individual or combined inhibition of CCL2, CCL5 and CCL9 however did not influence the numbers of lung monocytes in *Ccr2*^{*-*/*-*} mice 1 day after CpG treatment (Figures 7E and 7F), suggesting that splenic monocytes were attracted to the lung independently of those signals.

### Example 2: Therapeutic effect of CpG-induced interstitial macrophages in cigarette smoke-induced lung pathology

Since tobacco smoking is the most common cause of COPD, the present inventors investigated the role of CpG-induced interstitial macrophages in cigarette smoke-induced lung pathology.

Mice were exposed to cigarette smoke (50 cigarettes/day) for 3 days. One day later mice were sacrificed and the airway hyperreactivity (AHR) and IM numbers were assessed. For assessing the effects of CpG in the pathology, mice were instilled i.n. with 50 µl saline containing 50 µg non-CpG-DNA (ODN, Ref. HC4034, Sanbio) or 50 µg CpG-DNA (CpG, Ref. HC4033, Sanbio) 7 days before the first exposure (Figure 8A).

Airway hyperreactivity (AHR) was estimated by assessing dynamic airway resistance in anesthetized animals subjected to increasing doses of methacholine using a FlexiVent small animal ventilator (SCIREQ). Lung cell numbers were counted following whole lung digestion and mononuclear cell enrichment by the use of a hemocytometer. The numbers of regulatory macrophages were determined as CD45.2⁺ F4/80⁺ autofluorescent Ly-6C⁻ CD64⁺ cells.

Lung exposure to cigarette smoke triggered on the one hand an increase in airway hyperresponsiveness to methacholine (Figure 8B), and on the other hand a substantial decrease in IM numbers (Figure 8C). Moreover, pretreatment of such exposed mice with CpG was able to restore IM numbers to levels observed in non-exposed animals (Figure 8C), which was associated with an improvement in lung function (Figure 8B). Together, these data support that CpG-induced interstitial macrophages (IM_{CpG}) have a therapeutic, protective role in cigarette smoke-induced lung pathologies such as COPD.

### Example 3: Method for in vitro differentiation of monocytes to regulatory macrophages according to an embodiment of the present invention

Blood monocytes were sorted by FACS as follows. Blood was collected and mixed with EDTA (100 mM), and red blood cells were lysed with RBC lysis buffer (eBioscience). Cells were first enriched in CD11b-expressing cells by MACS using CD11b microbeads and then sorted based on their expression of CD45.2, CD115 and Ly-6C. Cell phenotyping and sorting were performed on a FACSCANTO II and a FACSARIA III (BD Biosciences), respectively. Staining reactions were performed at 4°C after incubation with 2.4G2 Fc receptor blocking antibodies (produced in house) to reduce non-specific binding. Regarding cell phenotyping and sorting, FSC-W and FSC-A discrimination was used to exclude doublet cells, and ViaProbe (7-AAD) cell viability solution (BD Biosciences) was used to discriminate between dead and living cells. Results were analyzed with FlowJo software (Tree Star).

Two hundred thousand (2x10⁵) blood monocytes (sorted by FACS) were cultured in RPMI medium supplemented with 10% FBS and additives (L-glutamine 8 mM, MEAA 0.1 mM, Pyruvate Na 1mM, Penicillin/streptomycin 50UI/ml, β-mercaptoethanol 0.5 mM), with or without CpG (7 µg/ml) (Ref. HC4033, Sanbio) in a 24 well plate. Twenty-four, 48 and 60 hours later, cells were harvested and phenotyped. ViaProbe (7-AAD) cell viability solution was used to discriminate between dead and living cells.

*Ex vivo* CpG treatment of blood monocytes promoted their survival and a differentiation pattern was observed from Ly-6C^{hi}CD64^{lo} (i.e. blood monocytes) to Ly-6C^{lo}CD64^{hi} cells, the phenotype of regulatory macrophages. The appearance of the Ly-6C/CD64 plot looked like a "waterfall" (arrows in Figure 9A), with the progressive acquisition of CD64 and the subsequent loss of Ly-6C (Figure 9B), showing that blood monocytes give rise to regulatory macrophages. Moreover, the Ly-6C^{lo}CD64^{hi} cells (i.e. the regulatory macrophages illustrating the invention) obtained from blood monocytes after 60h culture upon CpG stimulation produced IL-10 as quantified by ELISA (Figure 9C). Concentrations of IL-10 were measured by ELISA (eBioscience) in culture supernatants.

### Example 4: Methods for in vitro differentiation of monocytes to regulatory macrophages according to embodiments of the present invention

Regulatory macrophages are generated by *in vitro* differentiation of monocytes by culturing the monocytes in a medium in the presence of CpG.

The following sources of cells are tested from WT or IL-10-β-lactamase reporter (ITIB) mice: total bone marrow cells, FACS-sorted (CD45⁺Ly-6C⁺CD64⁻ or CD45⁺CD115⁺Ly-6C⁺CD64⁻ cells) monocytes from bone marrow, blood or spleen, and monocytes isolated from whole blood by density gradient centrifugation.

The following *in vitro* culture conditions are tested:
- **Basal culture medium:** RPMI medium (or any classical culture medium) supplemented with classical additives (L-glutamine 8 mM, MEAA 0.1 mM, Pyruvate Na 1mM, Penicillin/streptomycin 50UI ml-1, and/or β-mercaptoethanol 0.5 mM);
- **Serum:** different concentrations of heat-inactivated Fetal Bovine Serum, ranging from 0 to 10%, which may influence the differentiation potential and responsiveness of the regulatory macrophages;
- **Growth factors:** different concentrations of recombinant murine M-CSF, ranging from 0-100 ng/mL.
- **CpG nucleic acid treatment:** different concentrations, ranging from 0.1-20 µg/mL. CpG-DNA (Ref HC4033 from Sanbio) is used, or alternatively any other form of commercially available CpG-DNA;
- **Time of culture:** cells are analyzed at different time points, from 24 hours till 240 hours of culture including 24 hours (1 days), 36 hours, 48 hours (2 days), 56 hours, 60 hours, 72 hours (3 days), 96 hours (4 days), 120 hours (5 days), 144 hours (6 days), 168 hours (7 days), 192 hours (8 days), 216 hours (9 days), and 240 hours (10 days); and/or
- At the time of analysis, total cells, and/or non-adherent cells versus adherent cells are analyzed.

The assessment of survival and immunosuppressive functions *in vitro* of the regulatory macrophages is performed as follows:
- Survival is assessed by flow cytometry (7-AAD staining);
- IL-10 and TGF-beta levels at baseline or following stimulation with LPS or House Dust Mite (HDM) are measured in the supernatant of the culture; and/or
- in cells derived from ITIB mice, IL-10 expression is measured in total cells, adherent cells and/or non-adherent cells by flow cytometry staining.

### Example 5: Method for in vitro differentiation of monocytes to regulatory macrophages according to an embodiment of the present invention

In order to test whether regulatory macrophages cultured in the presence of an antigen before their collection and administration are capable to induce tolerance to the antigen in allergen-sensitized (asthmatic) mice, the following experiment is performed.

Monocytes are differentiated to regulatory macrophages by the methods as described in Example 4.

Prior to collection, the regulatory macrophages are cultured with an allergen (dust mites (HDM, Der f 1, or Der p 1), cat (Fel d 1), dog (Can f 1), or cockroach (Bla g1 or Bla g 2) or a combination thereof, for 6h, for 12h or for 24h. The regulatory macrophages are collected and administered to allergen-sensitized (asthmatic) mice in order to test the induction of tolerance to the allergen.

The *in vitro* differentiated allergen-pulsed regulatory macrophages obtained by a method according to an embodiment of the invention are administered by the intratracheal or the subcutaneous route. The regulatory macrophages are administered 1 to 5 times; weekly, bi-monthly or monthly.

The protective (tolerogenic) effects of the *in vitro* differentiated allergen-pulsed regulatory macrophages is assessed during a subsequent challenge. It is observed that allergen-sensitized mice treated with allergen-pulsed regulatory macrophages obtained by a method according to an embodiment of the invention are protected against the asthmatic manifestations upon a subsequent challenge. The mouse model of asthma (sensitized mice) is described in Example 6.

### Example 6: In vivo immunosuppressive functions and immunotherapeutic potential of in vitro differentiated regulatory macrophages according to an embodiment of the invention

*In vitro* or *ex vivo*-differentiated regulatory macrophages (M_{regs}) from IL-10-β-lactamase reporter (ITIB) CD45.2 mice (cell numbers ranging from 1.10⁵ to 10.10⁶ cells) are transferred by intratracheal (i.t.) instillation in the airways of naive WT CD45.1 animals. The transferred CD45.2 M_{regs} are followed over time (until 6 months) and their survival and IL-10 production is quantified by flow cytometry. Transferred mice are also challenged with House Dust Mite (HDM), a major allergen source in humans, and the survival and IL-10 production of CD45.2 M_{regs} is evaluated.

As show in Examples 1 and 2, adoptive transfer of *in vivo* CpG-induced IMs (IM_{CpG}) can recapitulate the protective effects of CpG nucleic acid on established asthma and cigarette smoke-induced lung pathology, respectively. In order to assess whether adoptive transfer of *in vitro* CpG-differentiated M_{regs} (cell numbers ranging from 1.10⁵ to 1.10⁷ cells) is able to ameliorate the pathology in a mouse model of HDM-induced asthma or in a mouse model of cigarette smoke-induced COPD, M_{regs} are transferred to a mouse model of HDM-induced asthma or a mouse model of COPD. Undifferentiated monocytes are used as controls in these transfer studies. Cells are transferred 1 to 60 days before the allergenic challenge.

Mouse model of HDM-induced asthma: Mice are sensitized by 2 weekly injections of 100 µg HDM or saline (Day 0 and day 7). At day 20, mice are transferred i.t. with M_{regs}. At days 21 to 80, mice are challenged with 100 µg HDM and sacrificed 3 days later for asthma assessment.

Mouse model of COPD: Mice are exposed to cigarette smoke generated from 5 cigarettes per day, 5 days a week, and up to 24 weeks (control groups are exposed to ambient air). Mice are transferred with M_{regs} each week, each month, each week from week 12 or 16, each two weeks from week 12 or 16, or each month from week 12 or 16. Mice are sacrificed the last day of smoke exposure and COPD is assessed.

## Claims

1. A method for *in vitro* differentiation of monocytes to regulatory macrophages, comprising culturing the monocytes in a medium in the presence of an immunostimulatory nucleic acid.

2. The method according to claim 1, comprising:
(a) isolating monocytes from a biological sample of a subject, thereby obtaining monocytes or a cell population comprising monocytes;
(b) adding the monocytes or the cell population comprising monocytes to a medium comprising an immunostimulatory nucleic acid, thereby obtaining a monocyte-medium mixture; and
(c) culturing the monocyte-medium mixture, thereby obtaining regulatory macrophages or a cell population comprising regulatory macrophages.

3. The method according to claim 1 or 2, wherein the monocytes are isolated from whole blood or bone marrow.

4. The method according to any one of claims 1 to 3, wherein the monocytes are circulating monocytes.

5. The method according to any one of claims 1 to 4, wherein the monocytes are human monocytes.

6. The method according to any one of claims 1 to 5, wherein the monocytes are classical monocytes, such as wherein the monocytes are CD14⁺⁺CD16⁻ cells.

7. The method according to any one of claims 1 to 6, wherein the concentration of monocytes in the medium is between about 1 x 10² and about 1 x 10⁹ cells per ml of the medium, such as wherein the concentration of monocytes in the medium is between about 1 x 10³ and about 1 x 10⁸, or between about 1 x 10⁴ and about 1 x 10⁷ cells per ml of the medium, or between about 1 x 10⁴ and about 1 x 10⁶ cells per ml of the medium, preferably wherein the concentration of monocytes in the medium is between about 5 x 10⁴ and about 2 x 10⁵ cells per ml of the medium.

8. The method according to any one of claims 1 to 7, wherein the immunostimulatory nucleic acid is a CpG nucleic acid.

9. The method according to any one of claims 1 to 8, wherein the immunostimulatory nucleic acid is a nucleic acid consisting of from 6 to 100 nucleotides, such as wherein the immunostimulatory nucleic acid is a nucleic acid consisting of from 6 to 50, from 6 to 40, from 6 to 30, from 8 to 50, from 8 to 40, from 8 to 30, from 10 to 50, from 10 to 40, from 10 to 30, from 15 to 30, or from 18 to 28 nucleotides.

10. The method according to any one of claims 1 to 9, wherein the concentration of the immunostimulatory nucleic acid in the medium is from 0.01 to 200 µg per ml of the medium, such as wherein the concentration of the immunostimulatory nucleic acid in the medium is from 0.025 to 175 µg/ml, from 0.050 to 150 µg/ml, from 0.075 to 125 µg/ml, from 0.10 to 100 µg/ml, from 0.25 to 75 µg/ml, from 0.50 to 50 µg/ml, or from 0.75 to 25 µg/ml of the medium, preferably wherein the concentration of the immunostimulatory nucleic acid in the medium is from 0.1 to 20 µg per ml of the medium.

11. The method according to any one of claims 1 to 10, wherein the monocytes or the cell population comprising monocytes are cultured in a medium in the presence of an immunostimulatory nucleic acid for 24 hours (1 day) to 240 hours (10 days), such as for 24 hours (1 day) to 192 hours (8 days), for 24 hours (1 day) to 144 hours (6 days), for 24 hours (1 day) to 96 hours (4 days), for 24 hours (1 day) to 72 hours (3 days), or for 48 hours (2 days) to 144 hours (6 days), for 48 hours (2 days) to 120 hours (5 days), for 48 hours (2 days) to 96 hours (4 days), or for 48 hours (2 days) to 72 hours (3 days).

12. The method according to any one of claims 1 to 11, wherein the method further comprises culturing the monocytes or the cell population comprising monocytes in the presence of macrophage colony-stimulating factor (M-CSF).

13. The method according to any one of claims 1 to 12, wherein the method further comprises culturing the regulatory macrophages or cell population comprising regulatory macrophages in the presence of one or more antigens prior to collection of the cells, preferably for 1 hour to 24 hours prior to collection of the cells.

14. Regulatory macrophages obtainable or obtained by a method as defined in any one of claims 1 to 13.

15. The regulatory macrophages according to claim 14, for use in treating an inflammatory respiratory disease.

16. The regulatory macrophages for use according to claim 15, wherein the inflammatory respiratory disease is selected from the group consisting of asthma, chronic obstructive pulmonary disease (COPD), chronic bronchitis, acute respiratory distress syndrome (ARDS), pneumonia, respiratory distress of prematurity, cystic fibrosis, pulmonary fibrosis, and pulmonary sarcoidosis.

17. The regulatory macrophages for use according to any one of claim 15 or 16, wherein the regulatory macrophages are to be administered to an autologous subject.

18. The regulatory macrophages for use according to any one of claims 15 to 17, wherein the regulatory macrophages are to be administered by intratracheal, intrabroncheal, or subcutaneous administration.

19. A pharmaceutical composition comprising the regulatory macrophages as defined in claim 14.
